# EUROPEAN PATENT APPLICATION

(11) **EP 1 895 468 A2**
(43) Date of publication of application: **05.03.2008**
(21) Application number: 07016864.6
(22) Date of filing: 28.08.2007
(51) Int. Cl.: G06T 7/00, G06Q 50/00, G06F 19/00

(54) **Medical image processing apparatus**

(30) Priority: 29.08.2006 JP 2006232412
(71) Applicant: KABUSHIKI KAISHA TOSHIBA, Tokyo (JP); Toshiba Medical Systems Corporation, Otawara-shi Tochigi (JP)
(72) Inventor: Tsubura, Shinichi, c/o IP Dpt, Otawara-shi Tochigi (JP)
(74) Representative: Kramer - Barske - Schmidtchen

(57) **Abstract**

Reference information such as medical-knowledge database 111 and statistical-information database 112 are stored in an information storage part 11 of a medical image processing apparatus 1. When a communication part 16 has accepted image data of a medical image and incidental information thereof from a modality 2000, an information changing part 12 compares this incidental information with the reference information stored in the information storage part 11, and changes medical information included in this incidental information. An information updating part 13 updates the statistical-information database 112 and so on based on a result of change of the incidental information. With this configuration, it becomes possible to automatically correct the incidental information.

## Description

### BACKGROUND OF THE INVENTION

### 1. Field of the Invention

The present invention relates to a medical image processing apparatus configured to process image data of medical images and information thereon.

### 2. Description of the Related Art

With recent developments in information technology, digitization of data management has made progress also in the medical field. In the field of medical images, standardization of the technology is currently underway to handle image data of medical images and incidental information thereof by using a standard referred to as DICOM (Digital Imaging and Communications in Medicine).

In a system pursuant to DICOM, categories (referred to as DICOM tags) of incidental information of image data are preset. When an order is issued, each piece of information included in the order is written into a predetermined category of incidental information to generate incidental information. In a case where the order includes information that is not preset as a DICOM tag, a new DICOM tag is additionally created, and the information is written therein.

Japanese Unexamined Patent Application Publication No.2004-171386 discloses one example of such technology. The technology described in JP-A 2004-171386 is for determining the adequacy of externally inputted information (incidental information) on an examination (X-ray imaging) and, when the information is determined to be inadequate, urging correction or calling attention.

However, the technology described in JP-A 2004-171386 requires a correction work to be manually performed by a user recognizing the information urging correction or calling attention. Therefore, the correction work is burdensome, and moreover, correction contents may be inputted incorrectly.

In addition, correction of order information has also been manually performed conventionally, so that the correction work has been troublesome and there has been a fear of inaccurate correction.

### SUMMARY OF THE INVENTION

An object of the present invention is to provide a medical image processing apparatus capable of automatically correcting incidental information of image data of a medical image.

Another object of the present invention is to provide a medical image processing apparatus capable of automatically correcting order information for capturing a medical image.

In a first aspect of the present invention, a medical image processing apparatus comprises: an accepting part configured to accept image data of a medical image and incidental information thereof; a determining part configured to compare predetermined reference information that includes associated information associating a plurality of medical information based on a degree of accuracy, with medical information included in the incidental information, and determine whether the medical information conforms to the predetermined reference information; and a changing part configured to change the medial information included in the incidental information based on the predetermined reference information when it is determined the medical information does not conform.

In the first aspect, the medical image processing apparatus acts so as to determine whether medical information included in incidental information of image data of a medical image conforms to predetermined reference information and, when determining not conform, change the medical information included in the incidental information. Accordingly, the medical image processing apparatus is capable of automatically correcting the incidental information.

In a second aspect of the present invention, a medical image processing apparatus comprises: an accepting part configured to accept order information for capturing a medical image; a storing part configured to store the order information accepted by the accepting part; and a changing part configured to, when the accepting part has accepted new order information, compare the new order information with past order information stored in the storing part, and change the new order information.

In the second aspect, the medical image processing apparatus acts so as to accept order information for capturing a medical image and stores the order information in a storing part and, when accepting new order information, compare the new order information with past order information stored in the storing part and change the new order information. Accordingly, the medical image processing apparatus is capable of automatically correcting the order information.

### BRIEF DESCRIPTION OF THE DRAWINGS

Fig. 1 is a schematic block diagram illustrating one example of an operating pattern of a medical system including a preferred embodiment of the medical image processing apparatus according to the present invention.
Fig. 2 is a schematic block diagram illustrating one example of a functional configuration of a preferred embodiment of the medical image processing apparatus according to the present invention.
Fig. 3 is a view illustrating one example of a pattern of examination-type statistical information stored in a preferred embodiment of the medical image processing apparatus according to the present invention.
Fig. 4 is a view illustrating one example of a pattern of imaged-sheet-number statistical information stored in a preferred embodiment of the medical image processing apparatus according to the present invention.
Fig. 5 is a view illustrating one example of a pattern of imaging-room statistical information stored in a preferred embodiment of the medical image processing apparatus according to the present invention.
Fig. 6 is a view illustrating one example of a pattern of order hierarchy information stored in a preferred embodiment of the medical image processing apparatus according to the present invention.
Fig. 7 is a view illustrating one example of a pattern of interpretation hierarchy information stored in a preferred embodiment of the medical image processing apparatus according to the present invention.
Fig. 8 is a schematic view illustrating one example of a lung-region schema template stored in a preferred embodiment of the medical image processing apparatus according to the present invention.
Fig. 9 is a schematic view illustrating one example of an upper-body-region schema template stored in a preferred embodiment of the medical image processing apparatus according to the present invention.
Fig. 10 is a schematic view illustrating one example of a schema included in order information inputted into a preferred embodiment of the medical image processing apparatus according to the present invention.
Fig. 11 is a schematic view illustrating one example of a schema included in order information inputted into a preferred embodiment of the medical image processing apparatus according to the present invention.
Fig. 12 is a schematic view illustrating one example of a pixel-value histogram generated by a preferred embodiment of the medical image processing apparatus according to the present invention.
Fig. 13 is a flowchart illustrating one example of an operation mode of a preferred embodiment of the medical image processing apparatus according to the present invention.
Fig. 14 is a flowchart illustrating one example of an operation mode of a preferred embodiment of the medical image processing apparatus according to the present invention.
Fig. 15 is a flowchart illustrating one example of an operation mode of a preferred embodiment of the medical image processing apparatus according to the present invention.
Fig. 16 is a flowchart illustrating one example of an operation mode of a preferred embodiment of the medical image processing apparatus according to the present invention.
Fig. 17 is a flowchart illustrating one example of an operation mode of a preferred embodiment of the medical image processing apparatus according to the present invention.
Fig. 18 is a flowchart illustrating one example of an operation mode of a preferred embodiment of the medical image processing apparatus according to the present invention.
Fig. 19 is a flowchart illustrating one example of an operation mode of a preferred embodiment of the medical image processing apparatus according to the present invention.
Fig. 20 is a flowchart illustrating one example of an operation mode of a preferred embodiment of the medical image processing apparatus according to the present invention.
Fig. 21 is a flowchart illustrating one example of an operation mode of a preferred embodiment of the medical image processing apparatus according to the present invention.
Fig. 22 is a view illustrating one example of a pattern of incidental information processed by an operation mode of a preferred embodiment of the medical image processing apparatus according to the present invention.
Fig. 23 is a view illustrating one example of a pattern of order information processed by an operation mode of a preferred embodiment of the medical image processing apparatus according to the present invention.
Fig. 24 is a view illustrating one example of a pattern of incidental information corrected by an operation mode of a preferred embodiment of the medical image processing apparatus according to the present invention.
Fig. 25 is a schematic view illustrating one example of a pattern of a background region of a medical image processed by an operation mode of a preferred embodiment of the medical image processing apparatus according to the present invention.
Fig. 26 is a schematic view illustrating one example of a pattern of a background region of a medical image processed by an operation mode of a preferred embodiment of the medical image processing apparatus according to the present invention.
Fig. 27 is a schematic view illustrating one example of a pattern of a background region of a medical image processed by an operation mode of a preferred embodiment of the medical image processing apparatus according to the present invention.
Fig. 28 is a schematic view illustrating one example of a pattern of a background region of a medical image processed by an operation mode of a preferred embodiment of the medical image processing apparatus according to the present invention.
Fig. 29 is a schematic block diagram illustrating one example of an operating pattern of a medical system including a preferred embodiment of the medical image processing apparatus according to the present invention.
Fig. 30 is a schematic block diagram illustrating one example of a functional configuration of a preferred embodiment of the medical image processing apparatus according to the present invention.
Fig. 31 is a flowchart illustrating one example of an operation mode of a preferred embodiment of the medical image processing apparatus according to the present invention.

### DETAILED DESCRIPTION OF THE PREFERRED EMBODIMENTS

One example of a preferred embodiment of the medical image processing apparatus according to the present invention will be explained in detail referring to the drawings.

### FIRST EMBODIMENT

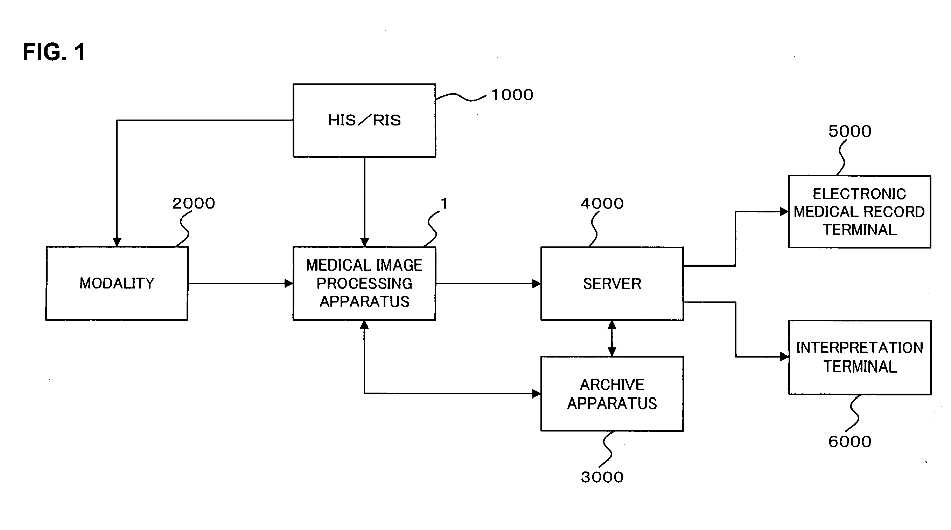

A first embodiment of the medical image processing apparatus according to the present invention will be explained here. Fig. 1 illustrates one example of the configuration of a medical system operated including the medical image processing apparatus according to this embodiment. This medical system comprises a medical image processing apparatus 1, an HIS/RIS 1000, a modality 2000, an archive apparatus 3000, a server 4000, an electronic medical record terminal 5000, and an interpretation terminal 6000. These apparatuses are connected via a communication line such as a LAN (Local Area Network) and a leased line.

Hereinafter, the HIS/RIS 1000, the modality 2000, the archive apparatus 3000, the server 4000, the electronic medical record terminal 5000 and the interpretation terminal 6000 that have the same configurations as conventional ones will be each briefly explained, and thereafter, the medical image processing apparatus 1 according to the present invention will be explained.

### HIS/RIS

The HIS/RIS 1000 is an information system that is an integration of an HIS (Hospital Information System) and an RIS (Radiology Information System).

The HIS is a computer system for efficiently performing services in a medical institution. The HIS typically includes various computer systems, such as an order-entry system for producing orders, a medical accounting system, a medication system, and a ward management system.

The RIS is a computer system for efficiently performing radiology services in a medical institution. The RIS typically performs a referring process of orders, a recording process and a transfer process of examination administration information and accounting information, various statistical processes, and so on.

The HIS/RIS 1000 transmits order information regarding a produced order to both the medical image processing apparatus 1 and the modality 2000.

### Modality

The modality 2000, which is referred to as a medical image diagnostic apparatus or the like, is an apparatus configured to detect data reflecting the internal geometry of a subject and image data of a medical image based on the detected data.

Further, the modality 2000 generates incidental information (of DICOM format) based on the order information transmitted from the HIS/RIS 1000. Then, the modality 2000 attaches the incidental information to the image data of the medical image, and transmits to the medical image processing apparatus 1.

As the modality 2000, any medical image diagnostic apparatus can be applied, such as an X-ray diagnostic apparatus, an X-ray CT (Computed Tomography) apparatus, an MRI (Magnetic Resonance Imaging) apparatus, a PET (Positron Emission Computed Tomography) apparatus, a SPECT (Single Photon Emission Computed Tomography) apparatus, and an ultrasonic diagnostic apparatus. The modality 2000 may be a medical imaging apparatus, such as an endoscope.

The X-ray diagnostic apparatus is, for example, a CR (Computed Radiography) apparatus, a DR (Digital Radiography) apparatus and a DA (Digital Angiography) apparatus. An X-ray diagnostic apparatus of a type capturing an image on an X-ray film is employed along with a digitizer for reading the image formed on the X-ray film. It is also possible to employ an X-ray diagnostic apparatus of a type forming digital data before creating an X-ray film.

The modality 2000 may create and output digital data of a medical image, or may output a medium such as a film. In the latter case, the modality 2000 shall comprise a part configured to convert an image formed on this medium into digital data.

Although only one modality 2000 is illustrated in Fig. 1, a plurality of modalities are used in general. Herein, it is possible to use a plurality of modalities of the same type (for example, two X-ray diagnostic apparatuses), and it is also possible to use different types of modalities (for example, an X-ray diagnostic apparatus and an X-ray CT apparatus). In a case where a plurality of modalities are used, each of the HIS/RIS 1000 and the medical image processing apparatus 1 will be communicatively connected to each of the modalities.

In the present invention, the apparatus configured to input the image data of medical images and the incidental information into the medical image processing apparatus 1 is not limited to the modality. For example, it is also possible to adopt a system configuration into which image data and so on are inputted from an external server or the like.

### Archive Apparatus

The archive apparatus 3000 is an apparatus configured to store various kinds of medical information such as image data of medical images, incidental information, and electronic medical-record data. The archive apparatus 3000 is, for example, a PACS (Picture Archiving and Communication System) that stores medical images and incidental information, an electronic medical record database, and the like. The archive apparatus 3000 obtains image data of medical images and incidental information via the medical image processing apparatus 1.

### Server

The server 4000 is an apparatus configured to distribute various kinds of medical information such as image data of medical images, incidental information and electronic medical-record data. The distributed information is, for example: image data of medical images and incidental information inputted from the medical image processing apparatus 1; and image data of medical images, incidental information and electronic medical record data stored in the archive apparatus 3000.

### Electronic Medical record terminal

The electronic medical record terminal 5000 is a terminal apparatus used to access or correct an electronic medical record. An accessing person or the like operates the electronic medical record terminal 5000, thereby requesting the server 4000 to distribute the electronic medical record of a desired patient. Upon receiving the request, the server 4000 distributes the data from the electronic medical record designated by the accessing person or the like to the electronic medical record terminal 5000. The electronic medical record terminal 5000 displays an electronic medical record based on the received data.

### Interpretation Terminal

The interpretation terminal 6000 is a terminal apparatus used in an operation of interpreting medical images, and is also referred to as an image viewer or the like. A doctor operates the interpretation terminal 6000, thereby requesting the server 4000 to distribute image data of a desired medical image or incidental information. Upon receiving the request, the server 4000 distributes the image data of the medical image or the incidental information designated by the doctor to the interpretation terminal 6000. The interpretation terminal 6000 displays a medical image or incidental information based on the received information.

### Medical Image processing apparatus

The medical image processing apparatus 1 is a computer configured to apply a process described later to image data of a medical image and incidental information generated by the modality 2000.

The medical image processing apparatus 1 has the same hardware configuration as a normal computer, and includes, for example, the following components: (1) a microprocessor such as a CPU (Central Processing Unit) for executing various control processes or arithmetic processes; (2) a main memory such as a RAM (Random-Access Memory); (3) an external storage unit such as a ROM (Read-Only Memory) and a hard disk drive; (4) a display device such as an LCD (Liquid-Crystal display) and a CRT (Cathode-Ray Tube); (5) an operating device and an input device, such as a keyboard, a mouse, a joy stick, a track ball, and a control panel; and (6) a communication interface such as a LAN card. These apparatuses (1) to (6) are connected via a bus or a cable.

Computer programs such as an application program for making the medical image processing apparatus 1 execute a process described later and an OS (Operating System) are previously stored in the above-described external storage unit (3). The microprocessor (1) executes an objective process by loading the computer program into the main memory (2).

The display device (4) and the operating device or the input device (5) are independently configured in the above hardware configuration example, but it is also possible to employ a pen tablet or touch-panel LCD in which both the devices are unified.

### Functional Configuration of Medical Image processing apparatus

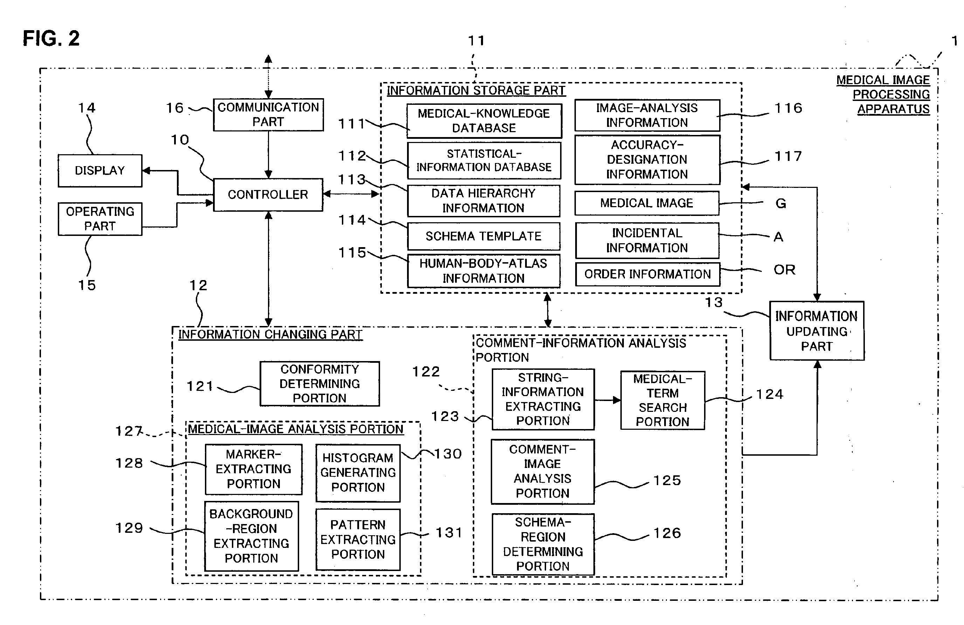

A functional configuration of the medical image processing apparatus 1 having the aforementioned hardware configuration will now be explained. A block diagram shown in Fig. 2 illustrates one example of the functional configuration of the medical image processing apparatus 1.

The medical image processing apparatus 1 comprises a controller 10, an information storage part 11, an information changing part 12, an information updating part 13, a display 14, an operating part 15, and a communication part 16. Hereinafter, these parts 10 to 16 will be each explained in detail.

### Controller

The controller 10 controls each part of the medical image processing apparatus 1. Specific contents of the control processes executed by the controller 10 will be explained below as necessary. The controller 10 includes (1) the microprocessor, (2) the main memory, and (3) the external storage unit in the above hardware configuration example.

### Information storage part

The information storage part 11 stores various types of information processed by the medical image processing apparatus 1. The information storage part 11 stores, for example, (image data of) a medical image G generated by the modality 2000 and incidental information A thereof, order information OR inputted from the HIS/RIS 1000, and so on. The information storage part 11 includes (2) the main memory and (3) the external storage unit of the above hardware configuration example.

Further, the information storage part 11 pre-stores information as described below: a medical-knowledge database (medical-knowledge DB) 111, a statistical-information database (DB) 112, data hierarchy information 113, a schema template 114, human-body-atlas information 115, image-analysis information 116, and accuracy-designation information 117. Hereinafter, the information 111 to 117 will be each explained.

### [Medical-knowledge database]

The medical-knowledge database 111 is a database that stores terms and systematic knowledge of the medical field, and so on. Specifically, the medical-knowledge database 111 stores the meanings of a large number of medical terms.

Herein, the medical-knowledge database 111 may searchably store the meaning of each medical term like a dictionary of medical terms, or may searchably store the meanings of medical terms included in a text or the like written as systematic knowledge, like a technical book of the medical field.

Further, the medical-knowledge database 111 may searchably store information (teaching file) of typical cases or unique cases to be used for study or education.

Furthermore, the medical-knowledge database 111 may include information such as the meanings of medical terms and texts representing characteristics of medical image capturing. This information may be one obtained either empirically or theoretically.

### [Statistical-information database]

The statistical-information database 112 is a database that stores statistical information regarding various types of medical information. This database can be created by, for example, accumulating clinical data in the medical institution.

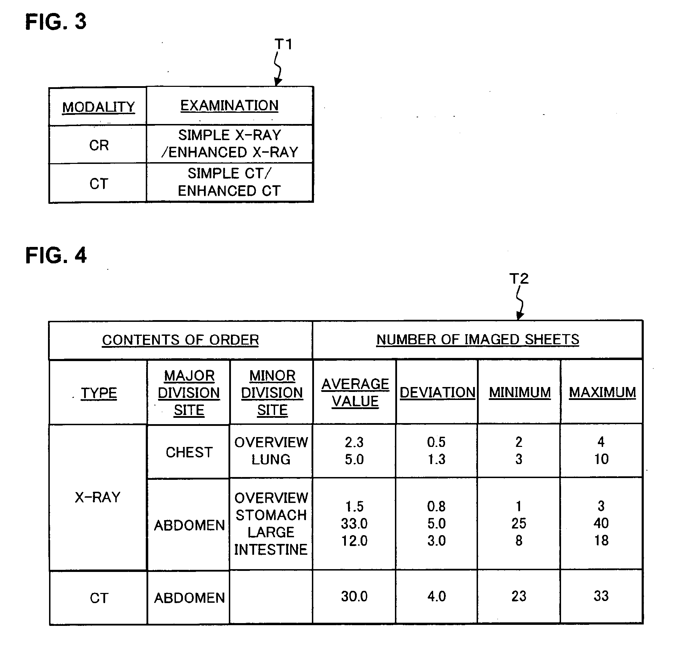

The information included in the statistical-information database 112 is employed as one example of "associated information" of the present invention. The associated information is information defining the association among medical information included in incidental information, and specifically, the association between high-accuracy information and low-accuracy information (described later). The statistical-information database 112 includes, for example, statistical information as shown in Figs. 3 to 5.

Examination-type statistical information T1 shown in Fig. 3 is one example of examination-type statistical information that associates a modality type with an examination type. This examination-type statistical information is information that associates each type of modality installed in the medical institution with what examination the modality is employed in (namely, what image the modality is employed to capture). It is possible to create the examination-type statistical information by recording, for each modality installed in the medical institution, what examination the modality has actually been employed in.

The examination-type statistical information T1 of Fig. 3 includes "CR (X-ray diagnostic apparatus)" and "CT (X-ray CT apparatus)" as modality types. Then, examination types "simple X-ray (X-ray imaging without a contrast agent)" and "enhanced X-ray (X-ray imaging with a contrast agent)" are associated with the modality type "CR." Meanwhile, examination types "simple CT (CT imaging without a contrast agent)" and "enhanced CT (CT imaging with a contrast agent)" are associated with the modality type "CT." The modality types and examination types thus associated can be identified in a process by the medical image processing apparatus 1.

Imaged-sheet-number statistical information T2 shown in Fig. 4 is one example of imaged-sheet-number statistical information that associates imaging-type information indicating the imaging type of a medical image in order information with statistical information indicating the number of imaged sheets of a medical image.

Herein, the imaging-type information is information indicating the type (imaging type) of an imaging pattern of a medical image, such as an examination type indicating an examination method (imaging method) like X-ray simple radiography and enhanced CT radiography, a modality type, and an imaged site. The examination type and the modality type are associated with each other in the examination-type statistical information T1 as described above.

It is possible to create this imaged-sheet-number statistical information by associating the imaging-type information and imaged-sheet-number information of the order information for actually performed imaging. At this moment, order information with the same contents are grouped (the order information do not need to have completely the same contents, and it is sufficient to group the order information in which predetermined contents are the same), and statistical information of the number of imaged sheets is created for each group.

In the imaged-sheet-number statistical information T2 of Fig. 4, combination of "type (imaging type)," "major division site (major division of an imaged site)," and "minor division site (minor division of an imaged site)" is considered as the contents of order information. Then, imaged-sheet-number statistical information including "average value," "deviation (standard deviation)," "minimum (minimum number of sheets)," and "maximum (maximum number of sheets)" are associated with each combination.

For example, in a case where the contents of order information are "X-ray (both the simple X-ray and the enhanced X-ray)," "chest" and "overview," that is, in a case where the overview of the chest is imaged with the X-ray diagnostic apparatus, the average number of the imaged sheets is 2.3, the standard deviation is 0.5, the minimum number of sheets is 2, and the maximum number of sheets is 4.

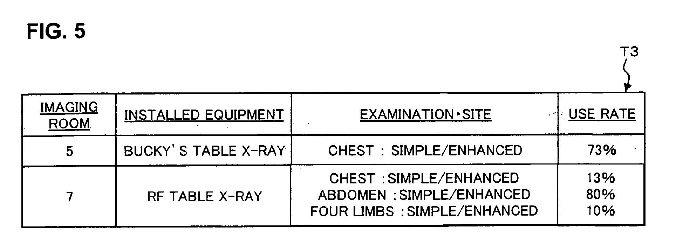

Imaging-room statistical information T3 shown in Fig. 5 is one example of imaging-room statistical information that associates imaging-type information indicating the imaging type of a medical image with statistical information indicating the frequency of use of an imaging room for capturing medical images. In other words, the imaging-room statistical information is information indicating how frequently each imaging room is used for each type of imaging.

For example, it is possible to create the imaging-room statistical information by, for imaging having been actually performed, associating the imaging type with the imaging room (identification information of the imaging room) obtained from the contents of the order information. At this moment, order information of the same imaging type are grouped, and statistical information of the frequency in use of the imaging room is created for each group.

The imaging-room statistical information T3 shown in Fig. 5 is (part of) the imaging-room statistical information regarding X-ray imaging. This imaging-room statistical information T3 has an "imaging room" section, an "installed equipment" section, an "examination and site" section, and a "use rate" section. In the "imaging room" section, identification information of the imaging rooms in the medical institution is recorded. In the "installed equipment" section, the type of the modality (X-ray diagnostic apparatus) installed in the imaging room is recorded. In the "examination and site" section, the imaged site and the examination type (either simple imaging or enhanced imaging) are recorded. In the "use rate" section, for imaging types specified by the recorded contents in the "installed equipment" section and the "examination and site" section, the use rate of each imaging room is recorded.

For example, for the imaging-type "X-ray imaging (both simple and enhanced), chest," the use rate in the imaging room "5" is 73%, and the use rate in the imaging room "7" is 13%. In other words, for this imaging type, it turns out that the frequency is much higher when the imaging is performed in the imaging room "5" than in the imaging room "7." The "X-ray imaging" in the imaging types can be obtained from the "Bucky's table X-ray" or the "RF table X-ray" that are the recorded contents of the "installed equipment" section.

### [Data hierarchy information]

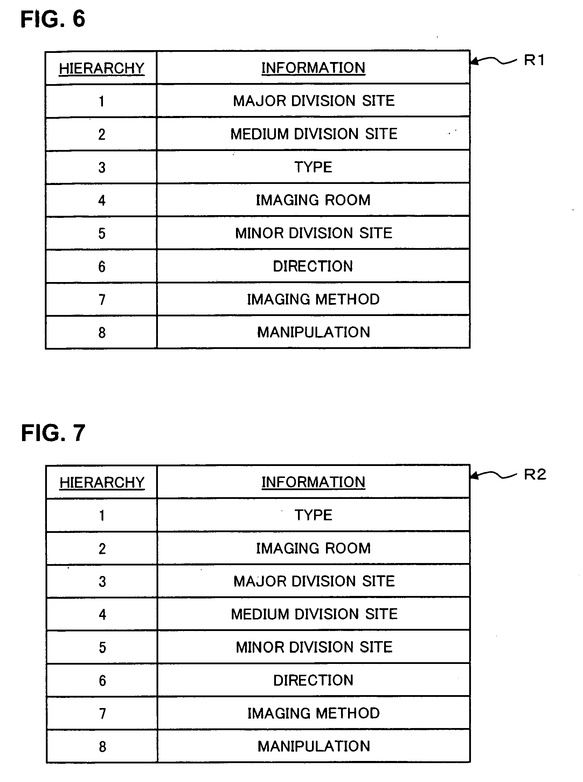

The data hierarchy information 113 stores a hierarchical structure of data to be processed by the medical image processing apparatus 1. The data hierarchy information 113 stores a pre-set hierarchical structure of data. In this hierarchical structure, at least one lower hierarchy is determined with respect to an upper hierarchy. Therefore, when information of a certain hierarchy is determined, it is possible to specify information that can appear in lower hierarchies than the certain hierarchy. In other words, when information of a certain hierarchy is determined, it is also possible to specify information that cannot appear in lower hierarchies than the certain hierarchy. The data hierarchy information 113 includes, for example, order hierarchy information R1 shown in Fig. 6 and interpretation hierarchy information R2 shown in Fig. 7.

The order hierarchy information R1 represents one example of a hierarchical structure of data included in order information, and defines a hierarchical structure that has been set with an objective of facilitating a work for producing an order. The data included in this order hierarchy information R1 has a structure composed of eight hierarchies described below: (1) top-level hierarchy (hierarchy 1) = "major division site (major division of imaged sites)," for example, chest, abdomen, and the four limbs; (2) second hierarchy (hierarchy 2) = "medium division site (medium division of imaged sites)," for example, an organ, an organ and periphery thereof, and a plurality of organs; (3) third hierarchy (hierarchy 3) = "type (modality type or examination type)," a modality type such as an X-ray diagnostic apparatus and an X-ray CT apparatus, and an examination type such as simple X-ray and enhanced CT; (4) fourth hierarchy (hierarchy 4) = "imaging room (identification information of an imaging room)"; (5) fifth hierarchy (hierarchy 5) = "minor division site (minor division of imaged sites)," for example, an organ, and a portion of an organ; (6) sixth hierarchy = "direction (imaging direction)," for example, the front side, the lateral side, and the upper side; (7) seventh hierarchy (hierarchy 7) = "imaging method," for example, Stenvers method; and (8) eighth hierarchy (hierarchy 8) = "manipulation," for example, catheters.

The interpretation hierarchy information R2 illustrates one example of a hierarchical structure of data subjected to interpretation of medical images, and defines a hierarchical structure that has been set for the purpose of facilitating an interpretation work. The data included in this interpretation hierarchy information R2 has a structure composed of eight hierarchies described below: (1) top-level hierarchy (hierarchy 1) = "type"; (2) second hierarchy (hierarchy 2) = "imaging room"; (3) third hierarchy (hierarchy 3) = "major division site"; (4) fourth hierarchy (hierarchy 4) = "medium division site"; (5) fifth hierarchy (hierarchy 5) = "minor division site"; (6) sixth hierarchy = "direction"; (7) seventh hierarchy (hierarchy 7) = "imaging method"; and (8) eighth hierarchy (hierarchy 8) = "manipulation."

Referring to the order hierarchy information R1 and the interpretation hierarchy information R2 as described above, in a case where "chest" is recorded in the major division site of the order information and "stomach" is recorded in the medium division site (or minor division site) of a lower level, the latter turns out to be an error (because "stomach" never appears in a lower level than "chest" and it should appear in a lower level than "abdomen.")

### [Schema template]

The schema template 114 is a template (image) of a schematic view of part of a human body employed in a schema (also referred to as punch drawing) for writing image information (such as a graphic specifying an affected area) into. As this schema template 114, it is possible to employ a template image of a schema used in production of an order or in creation of an electronic medical record.

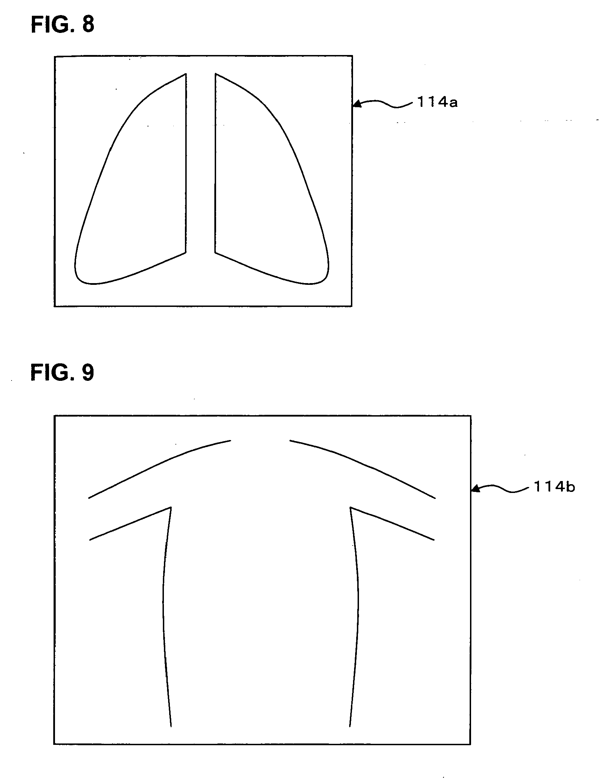

The schema template 114 includes, for example, a lung-region schema template 114a shown in Fig. 8, an upper-body-region schema template 114b shown in Fig. 9, and so on. The lung-region schema template 114a is a template image of a schematic view of a lung of a human body viewed from the front (or the back) side. The upper-body-region schema template 114b is a template screen of a schematic view of an upper portion of a human body viewed from the front (or the back) side.

### [Human-body-atlas information]

Human-body-atlas information 115 is image data of an anatomical drawing of a human body. This human-body-atlas information 115 can be obtained, for example, from the electronic medical record information in the archive apparatus 3000. The anatomical drawing based on this human-body-atlas information 115 is an anatomical drawing of an entire human body, and is an image in which a pattern of sites such as organs is depicted.

### [Image-analysis information]

Image-analysis information 116 includes various types of information regarding image analysis. For example, the image-analysis information 116 includes information subjected to an analysis process of image data of a medical image.

Information for this medical image analysis process includes, for example: (1) L-R mark pattern information that represents a pattern (shape, size, and so on) of an L-R mark indicating the horizontal direction of a medical image; (2) marker pattern information that represents a pattern of a marker indicating the reference position, the attention position, and so on, of a medical image; (3) threshold information of a pixel value for extracting a background region (an image region corresponding to a region where an X-ray has transmitted at the time of X-ray imaging) of a medical image; (4) histogram-associated information that associates a pattern of a pixel-value histogram (described later) illustrating the frequency for each pixel value of pixels that compose a medical image with a human body site (and the imaging direction thereof); and (5) a human-body-site template illustrating the shape of a captured image of a human body site.

Herein, it is common to use a uniform pattern of (1) the L-R mark and (2) the marker in the medical institution. Regarding the L-R mark, it is general to embed a letter "L" or "R" in a medical image.

In addition, the pixel-value histogram of (4) is obtained by counting, for each pixel value, the number of pixels having the pixel value to form a histogram. In general, the pixel-value histogram takes pixel values on the horizontal axis and the number of pixels on the vertical axis. This pixel-value histogram is known to have a characteristic pattern reflecting a human body site (imaged site) and an imaging direction. The histogram-associated information defines association between the pattern of the pixel-value histogram and the imaged site and imaging direction, by making use of this known relationship.

In addition, the human-body-site template (5) is image information having a general shape of an image region corresponding to a human body site such as a lung, a stomach, a chest and an abdomen, in the medical image obtained by imaging that human body site.
Moreover, the image-analysis information 116 includes information such as mathematical formulae and parameters for applying various approaches of image analysis. For example, information on an image-analytical approach such as KL (Karhumen-Lo'eve) transform, classification, AI (Artificial Intelligence) and texture analysis is included in the image-analysis information 116.

### [Accuracy-designation information]

Accuracy-designation information 117 is information that defines the accuracy of various types of medical information included in incidental information attached to image data of a medical image. The accuracy of the medical information is pre-designated based on an operating pattern of that medical system, a motion pattern of apparatuses such as the HIS/RIS 1000 and the modality 2000, and so on.

For example, considering the motion pattern of the modality 2000, there is only a relatively small possibility of incorrectly recording the number of imaged sheets of medical images into the incidental information, and therefore, the accuracy of the imaged-sheet-number information in the incidental information is relatively high. Moreover, there is only a relatively small possibility of mistaking the modality type, and therefore, the accuracy of the modality-type information in the incidental information is relatively high. The accuracy of imaged-site information and imaging method information in the incidental information is relatively low.

From such a viewpoint, the accuracy-designation information 117 is information that designates the accuracy of medical information included in incidental information. As one example, in a case where information on modality type, imaged site, imaging method, number of imaged sheets, imaging equipment, imaging room and so on are included in the incidental information, the information on the modality type, the examination type, the number of imaged sheets, the imaging equipment, the imaging room and so on are designated as high-accuracy information, and the information on the imaged site, the imaging method and so on, are designated as low-accuracy information.

### Information changing part

The information changing part 12 executes a process of changing incidental information attached to image data of a medical image. The information changing part 12 functions as one example of a "changing part" of the present invention. This information changing part 12 includes (1) the microprocessor, (2) the main memory, and (3) the external storage unit in the above hardware configuration example. The information changing part 12 includes a conformity determining portion 121, a comment-information analysis portion 122, and a medical-image analysis portion 127. Hereinafter, these parts will be each explained.

### [Conformity determining portion]

The conformity determining portion 121 executes a process of determining whether medical information included in incidental information conforms to statistical information (associated information) of the statistical-information database 112. The conformity determining portion 121 functions as one example of a "determining part" of the present invention. Hereinafter, a specific example of the determining process by this conformity determining portion 121 will be explained.

As a first specific example, a determining process of determining conforming/nonconforming to the imaged-sheet-number statistical information T2 (refer to Fig. 4) in the statistical-information database 112 will be explained. The imaged-sheet-number statistical information T2 is information that associates imaging-type information (such as examination type and imaged site) in order information with the statistical information of the number of imaged sheets, as described above.

Assuming the examination type and imaged site specified from the incidental information are "X-ray" and "chest, overview," in a case where the number of imaged sheets specified from the incidental information is "3," the conformity determining portion 121 refers to the statistical information of the number of imaged sheets corresponding to the "X-ray" and "chest, overview" in the imaged-sheet-number statistical information T2. In this statistical information, average value "2.3," deviation "0.5," minimum "2" and maximum "4" are recorded.

The conformity determining portion 121 determines the conformity by comparing the number of imaged sheets "3" in the incidental information and that statistical information. For example, the conformity determining portion 121 determines whether the number of imaged sheets "3" in the incidental information is included between the minimum "2" and the maximum "4" in the statistical information (in this case, "included"), thereby determining it "conforms."

In a case where the number of imaged sheets in the incidental information is, for example, "20," it is determined that it is not included between the minimum and the maximum in the statistical information. Furthermore, the conformity determining portion 121 calculates the probability that the number of imaged sheets becomes "20" based on the average value and deviation in the statistical information, and determines whether this probability is a predetermined value (e.g. 10%) or less. Then, the conformity determining portion 121 determines it to be "nonconforming" when this probability is measured to be equal to or less than the predetermined value. When the number of imaged sheets is 20, it will be determined to be "nonconforming." Meanwhile, when the probability is more than the predetermined value, it is determined to be "conforming."

Next, as a second specific example, a determining process of determining conformity/nonconformity to the imaging-room statistical information T3 (refer to Fig. 5) of the statistical-information database 112 will be explained. The imaging-room statistical information T3 is, as described above, information that associates the imaging-type information (examination type, imaged site, and so on) in the order information with the statistical information of the frequency of use of the imaging room.

Considering the examination type specified from the incidental information is "X-ray (simple/enhanced)" and the imaged site is "chest," in a case where (the identification information of) the imaging room specified from the incidental information is "5," the conformity determining portion 121 refers to the imaging-room statistical information T3 to obtain a use rate of "73%" for the imaging room corresponding to the imaging type. Further, the conformity determining portion 121 measures whether this use rate is more than a predetermined value (e.g. 60 %). The conformity determining portion 121 determines "conforming" when the use rate is more than the predetermined value, and determines "nonconforming" when the use rate is equal to or less than the predetermined value.

Meanwhile, in a case where the imaging room specified from the incidental information is "7," the conformity determining portion 121 obtains a corresponding use rate "13%," thereby determining "nonconforming."

In a case where there exist a plurality of imaging rooms falling under the imaging-type specified from the incidental information, the conformity determining portion 121 may determine conformity by comparing the use rates of the plurality of imaging rooms. For example, the conformity determining portion 121 may be configured so as to determine only the imaging room with the maximum use rate among the relevant plurality of imaging rooms to be "conforming."

### [Comment-information analysis portion]

The comment-information analysis portion 122 determines whether comment information is included in the order information and, when determining that comment information is included, analyzes the comment information. The comment-information analysis portion 122 functions as one example of a "comment-information analysis portion" of the present invention.

Herein, the comment information refers to various kinds of comments inputted when orders are produced. For example, the comment information is string information (a string comment) for describing a chief complaint and an attention site, and image information (an image comment) such as a schema. An example of the string comment is: "Focused on imaging of the left shoulder because the patient reported the pain of the left shoulder due to a traffic accident."

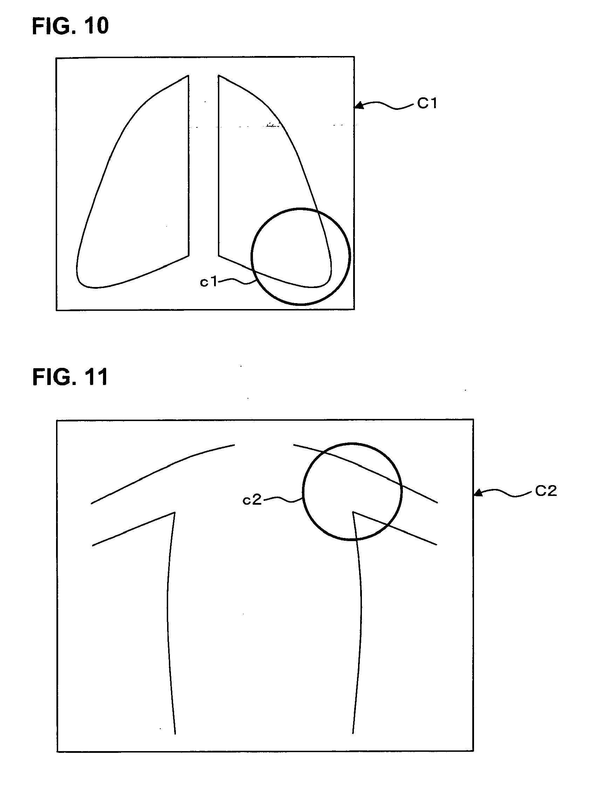

Figs. 10 and 11 illustrate one example of a schema as comment information (an image comment). A schema C1 shown in Fig. 10 is the lung-region schema template 114a of Fig. 8 on which image information c1 such as a graphic is written. Moreover, a schema C2 shown in Fig. 11 is the upper-body-region schema template 114b of Fig. 9 on which image information c2 is written.

When the comment information as described above is inputted when an order is produced, the HIS/RIS 1000 creates order information including the inputted comment information, and transmits to the modality 2000 and the medical image processing apparatus 1.

The comment-information analysis portion 122 includes a string-information extracting portion 123 and medical-term search portion 124 that operate in conjunction with each other, a comment-image analysis portion 125, and a schema-region determining portion 126. Hereinafter, these portions will be each explained.

### [String-information extracting portion]

The string-information extracting portion 123 executes a process of extracting a predetermined string from the string comment included in the order information. In specific, the string-information extracting portion 123 extracts strings equivalent to various medical terms stored in the medical-knowledge database 111, from the string comment.

For example, from the abovementioned string comment " Focused on imaging of the left shoulder because the patient reported the pain of the left shoulder due to a traffic accident," the string-information extracting portion 123 extracts strings (terms) such as "left shoulder," "pain, " and " imaging."

The string-extracting process can be executed by any known string-matching technique as necessary.

### [Medical-term search portion]

The medical-term search portion 124 operates in conjunction with the string-information extracting portion 123, and searches the meaning of the string (specifically, medical term) extracted from the string comment, in the medical-knowledge database 111. The medical-knowledge database 111 searchably stores meanings of medical terms as described above. Such a searching process can be executed by any known information-searching technique as necessary.

Further, the medical-term search portion 124 is capable of obtaining, based on information (both empirical information and theoretical information) illustrating characteristics of medical image capturing stored in the medical-knowledge database 111, characteristic information of image capturing based on the order information. For example, the medical-term search portion 124 is capable of obtaining a fact that a large number of images (e.g. several tens of images) will be captured, from a part "Focused on imaging of the left shoulder" included in the aforementioned string comment.

Furthermore, when the string-information extracting portion 123 has extracted an imaged site, imaging method and so on, the medical-term search portion 124 can obtain empirical information on the range of the number of imaged sheets according to the imaged site and imaging method, information on the imaging equipment, and so on.

### [Comment-image analysis portion]

The comment-image analysis portion 125 applies image analysis to a schema (image information) included in the order information. Specifically, the comment-image analysis portion 125 reads out the schema template 114 stored in the information storage part 11, and executes a process of calculating a subtraction image between the schema template 114 and the schema included in the order information. At this moment, the comment-image analysis portion 125 specifies the type of the schema (a site, direction, and so on), based on the description contents of the order information, such as the examination type, imaged site (at least one of the major division site, medium division site and minor division site) and the imaging method. An appropriate template is searched out from the schema template 114.

A specific example of a process executed by the comment-image analysis portion 125 will be explained. When the schema C1 shown in Fig. 10 is included in the order information, the comment-image analysis portion 125 specifies, based on the description contents of the order information, that the schema C1 is a schema of a lung region (chest) taken from the front.

Next, the comment-image analysis portion 125 searches out a template conforming to the type of the specified schema (that is, the lung-region schema template 114a shown in Fig. 8) from the schema template 114.

Furthermore, the comment-image analysis portion 125 calculates a subtraction image between the schema C1 and the lung-region schema template 114a. As a result, the image information c1 written in the schema C1 is extracted.

Herein, each of the schema C1 and the lung-region schema template 114a is a bi-level image. Therefore, it is possible to obtain the image information c1 by calculating the difference without making any changes (after matching image sizes, if necessary). In a case where either or both are not bi-level images, the difference may be calculated after any known operation for binary-image processing is applied.

In a case where the schema C2 shown in Fig. 11 is included in the order information, in a similar manner, the comment-image analysis portion 125 searches for the upper-body-region schema template 114b (refer to Fig. 9) and calculates a subtraction image between the schema C2 and the upper-body-region schema template 114b to extract the image information c2.

The image information written into the schema may include, other than a graphic such as the above-described image information c1 and c2a, a string manually inputted by a writer. In this case, the comment-image analysis portion 125 generates a subtraction image to extract the string, and recognizes the string by a known pattern-recognition technique.

Furthermore, the comment-image analysis portion 125 searches out the meaning of the string (specifically medical terms) from the medical-knowledge database 111, in the same manner as the medical-term search portion 124. The information thus searched out can be handled in a similar fashion to the information searched out by the medical-term search portion 124.

### [Schema-region determining portion]

The schema-region determining portion 126 executes a process of determining a region on an anatomical drawing of a human body equivalent to the schema included in the order information.

More specifically, the schema-region determining portion 126 first reads out the human-body-atlas information 115 from the information storage part 11. Next, the schema-region determining portion 126 specifies a site, direction and so on of the schema, based on description contents of the order information, such as the examination type, the imaged site and the imaging method. It is also possible to configure the schema-region determining portion 126 so as to record information on the site, direction and so on of the schema into the order information and specify the site and information of the schema based on the recorded information.

Furthermore, the schema-region determining portion 126 specifies a region on an anatomical drawing of a human body corresponding to the specified region. Then, the schema-region determining portion 126 determines the region equivalent to the schema on the human body anatomical drawing by executing an image alignment process, if necessary.

It is also possible to configure so that, at this moment, the controller 10 causes the display 14 to display the human body anatomical drawing and the schema overlapped with each other and the user operates the operating part 15 to move the schema on the human body anatomical drawing and perform the alignment.

### [Medical-image analysis portion]

The medical-image analysis portion 127 executes a process of generating predetermined reference information by analyzing the image data of a medical image. The medical-image analysis portion 127 functions as one example of an "image analysis portion" of the present invention.

The medical-image analysis portion 127 is provided with a marker-extracting portion 128, a background-region extracting portion 129, a histogram generating portion 130, and a pattern extracting portion 131. Hereinafter, these portions will be each explained.

### [Marker-extracting portion]

The marker-extracting portion 128 analyzes image data of a medical image and extracts a marker image in the medical image. Marker images to be extracted are an L-R mark indicating the horizontal direction of an image, and various kinds of markers indicating a reference position, attention position and so on in an image. The extracted marker image is employed as the above-mentioned reference information.

A specific example of a process by the marker-extracting portion 128 will be explained. The image-analysis information 116 stored on the information storage part 11 includes L-R-mark-pattern information that represents the pattern of the L-R mark and marker pattern information that represents the pattern of a marker, as described above. First, the marker-extracting portion 128 obtains the L-R-mark-pattern information and marker-pattern information (collectively referred to as "pattern information") from the image-analysis information 116. Next, the marker-extracting portion 128 analyzes, for each pattern information, image data of the medical image to search for a marker image conforming to the pattern information. Consequently, the marker image in the medical image is extracted.

In cases such as when a marker image is frequently embedded in (almost) the same position in a medical image, it is desirable to shorten the processing time by searching for the marker image from the near-field region of the embedded position.

In addition, a marker image typically has a size different from that in the pattern information, due to the zooming in and zooming out of the magnification ratio when imaging or displaying a medical image. The marker-extracting portion 128 matches the size of the marker image and that of the pattern information based on this magnification ratio.

Moreover, there are also cases in which a marker image is embedded in a medical image in an orientation different from the pattern information. In order to deal with such cases, the marker-extracting portion 128 extracts the objective marker image by changing the orientation of (rotating) the pattern information or marker image.

Similarly, there are also cases in which a marker image is embedded in a medical image in an inverted state. In order to deal with this case, the marker-extracting portion 128 extracts the objective marker image by inverting the pattern information or marker image.

The marker-image-extracting process as described above can be accomplished, for example, by employing the image-alignment process using a correlation coefficient.

### [Background-region extracting portion]

The background-region extracting portion 129 analyzes image data of a medical image, and extracts a background region from the medical image. The background region is an image region equivalent to a region where an X-ray has transmitted directly in the medical image obtained by X-ray imaging (i.e. a region where the X-ray has been detected without transmitting a human body). The extracted background region is employed as reference information.

A specific example of a process by the background-region extracting portion 129 will be explained. The background-region extracting portion 129 first obtains the threshold information of pixel values from the image-analysis information 116 stored on the information storage part 11. Next, the background-region extracting portion 129 compares the pixel values of each pixel composing the medical image with the threshold indicated in the threshold information, and extracts pixels with pixel values equal to or greater than this threshold. The pixels extracted by this process are pixels composing the background region.

Incidentally, considering that the radiographed region of interest is frequently disposed on the center of a medical image, it is also possible to extract a background region by extracting pixels with pixel values greatly different from the pixel values of pixels in the center.

In addition, in the pixel-value histogram to be described later, the background region may also be extracted from the pixel-value histogram by making use of the fact that pixels with pixel values equal to or greater than a predetermined value correspond to the background region.

### [Histogram generating portion]

The histogram generating portion 130 analyzes image data of a medical image and executes a process of generating a pixel-value histogram illustrating the frequency of each pixel value in a medical image. The generated pixel-value histogram is employed as reference information.

A specific example of a process by the histogram generating portion 130 will be explained. The histogram generating portion 130 obtains pixel values of respective pixels composing a medical image, and also counts the number of the pixels for each of the pixel values. Then, the histogram generating portion 130 generates a desired pixel-value histogram by plotting the results of counting the number of the pixels for each of the pixel values on a two-dimensional coordinate plane taking the pixel values on the horizontal axis and the number of pixels (frequency) on the vertical axis, and forming a line graph, a curve graph, a histogram or the like, from the plotted results.

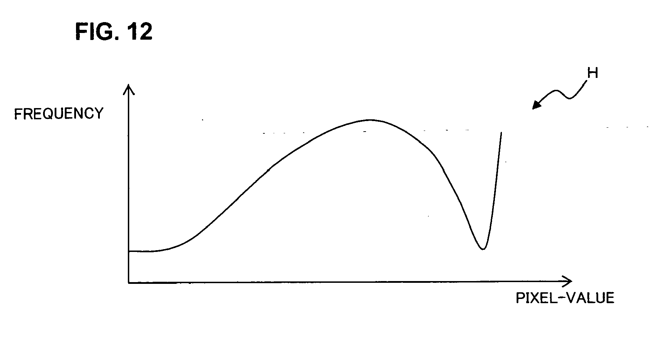

Fig. 12 illustrates one example of a pixel-value histogram generated by the histogram generating portion 130. A pixel-value histogram H shown in Fig. 12 is an example of a pixel-value histogram derived from image data of a medical image that has been obtained by imaging the chest of a subject from the front. The peak of a portion with a large pixel value in this pixel-value histogram H is equivalent to the background region of the medical image.

### [Pattern extracting portion]

The pattern extracting portion 131 analyzes image data of a medical image, and executes a process of extracting a region corresponding to part of a human body in the medical image. The extracted image region is employed as reference information.

A specific example of a process by the pattern extracting portion 131 will be explained. Firstly, the pattern extracting portion 131 obtains the abovementioned human-body-site template from the image-analysis information 116 stored in the information storage part 11. This human-body-site template is image information illustrating a captured image of a human body site in a general shape. For example, a human-body-site template of the lung is image information with a similar shape to the lung-region schema template 114a shown in Fig. 8. After obtaining the human-body-site template, the pattern extracting portion 131 extracts an image region that fits into the shape indicated in this human-body-site template, from the medical image. At this moment, the image region to be extracted has a shape different from that of the human-body-site template in general, but fits into the characteristic shape indicated in the human-body-site template. For example, the image region equivalent to the lung in a medical image of a chest (frontal view) has a shape different from the human-body-site template of the lung, but their characteristic shapes (such as the shape of the right and left lungs, the fact that they have almost symmetric shapes, and so on) fit into each other. The pattern extracting portion 131 extracts the objective image region by, for example, applying an image alignment process employing a correlation coefficient.

The pattern extracting portion 131 executes the above-described image-region-extracting process for the respective human-body-site templates included in the image-analysis information 116. As a result, the templates are divided into a human-body-site template from which the corresponding image region is extracted and a human-body-site template from which the corresponding image region is not extracted (the corresponding image region may be extracted from all of the templates, or may be extracted from none of the templates).

For example, from a medical image of the chest taken from the front, an image region corresponding to the lung is extracted, but an image region corresponding to the stomach is not extracted. Further, from a medical image of the upper body taken from the front, both the image region corresponding to the lung and the image region corresponding to the stomach are extracted.

### Information updating part

When the content of incidental information is changed by the information changing part 12, the information updating part 13 executes a process of updating statistical information in the statistical-information database 112, based on the result of the change. The information updating part 13 functions as one example of an "updating part" of the present invention.

A specific example of a process by the information updating part 13 will be explained. Firstly, a process of updating the examination-type statistical information T1 will be explained. When the information changing part 12 changes the content of the examination type and/or modality type in incidental information, the information updating part 13 obtains information of the changed modality type and examination type, and also obtains the examination-type statistical information T1 from the information storage part 11.

Next, the information updating part 13 determines whether a correspondence relationship between the changed modality type and examination type is included in the examination-type statistical information T1. When determining the correspondence relationship after change is included, the information updating part 13 stores the examination-type statistical information T1 in the information storage part 11 without updating. On the other hand, when determining the correspondence relationship after change is not included, the information updating part 13 adds the correspondence relationship after change into the examination-type statistical information T1 to update the information, and stores the updated examination-type statistical information T1 in the information storage part 11.

A process of updating the imaged-sheet-number statistical information T2 will be explained. When the information changing part 12 changes the contents of the examination type and imaged site in incidental information, the information updating part 13 obtains the changed contents and the information on the imaged-sheet-number in the incidental information. Further, the information updating part 13 obtains the imaged-sheet-number statistical information T2 from the information storage part 11.

Then, the information updating part 13 updates description contents in the "average," "deviation," "minimum," and "maximum" sections in the imaged-sheet-number statistical information T2, by reflecting those changes. For the "average" and "deviation" sections, the changed contents are added to the populations of the description contents before updating, the average value and standard deviation are calculated, and the description contents are changed to the calculation results. On the other hand, for the "minimum" and "maximum" sections, the minimum value and maximum value in the populations into which the changed contents are added are calculated, respectively, and the description contents are changed when the new minimum value or maximum value are different from those before updating. The description contents may be updated by comparing the value of the imaged-sheet-number indicated in the changed contents with the values described in the "minimum" or "maximum" sections before updating and calculating the magnitude relationship between them.

A process of updating the imaging-room statistical information T3 will be explained. When the information changing part 12 changes the contents of the examination type and imaged site in incidental information, the information updating part 13 obtains the changed contents and the identification information of an imaging room in the incidental information, and at the same time, obtains the imaging-room statistical information T3 from the information storage part 11.

Next, the information updating part 13 searches out the imaging type corresponding to the changed contents (description contents in the "examination and site" section) from the imaging-room statistical information T3, and specifies (identification information of) the imaging room corresponding to the searched imaging type. When only one imaging room is searched out, the information updating part 13 stores the imaging-room statistical information T3 on the information storage part 11 without updating.

Meanwhile, when two or more imaging rooms are searched out, the information updating part 13 specifies one of these imaging rooms based on the identification information of imaging rooms from the information changing part 12. Then, the use rate is calculated by adding "1" to the number of examinations performed in the specified imaging room, and the imaging-room statistical information T3 is updated and then stored in the information storage part 11.

A specific example of the case in which two or more imaging rooms have been sought will be explained. When the imaging type corresponding to the changed contents is "chest: simple," it is found that two or more rooms exist as an imaging room corresponding to the imaging type (imaging rooms "5," "7," and others (e.g. "12") by referring to the imaging-room statistical information T3.

Herein, assuming that the cumulative number of examinations performed of that imaging type before updating is 100, the examination was performed 73 times in the imaging room "5," 13 times in the imaging room "7," and 14 times in the imaging room "12," as can be seen from the imaging-room statistical information T3.

The information updating part 13 specifies, based on the identification information of the imaging room from the information changing part 12, the imaging room where that examination has been performed from among these imaging rooms. Assuming that the specified imaging room is "5," the cumulative number of times the examination was performed is 101, in which the examination was performed 73 + 1 = 74 times in the imaging room "5," 13 + 0 = 13 times in the imaging room "7," and 14 + 0 = 14 times in the imaging room "12."

The information updating part 13 can calculate a new use rate for the each of the imaging rooms for that imaging type by dividing a new number of times performed for each of the imaging rooms by a new cumulative number of times performed. In other words, the new use rates for the imaging rooms "5," "7," and "12" can be calculated with the following expressions, respectively: 74 ÷ 101, 13 ÷ 101, and 14 ÷ 101. The information updating part 13 updates the description contents in the "use rate" section in the imaging-room statistical information T3 with these new values of the use rates.

The process by the information updating part 13 described above is executed in accordance with changes made in incidental information. However, for example, it is also possible to configure so as to, when order information is inputted from the HIS/RIS 1000 to the medical image processing apparatus 1, execute the same updating process as described above, based on the contents of the order information. The information changing part 12 executes the changing process so as to coordinate the contents of incidental information with the contents of order information, so that the statistical-information database 112 is updated in the same manner no matter which updating process is applied.

### Display

The display 14 includes a display device such as the abovementioned LCD and CRT. This display 14 performs a display operation upon receiving a command from the controller 10.

### Operating part

The operating part 15 includes an operating device or input device, such as the abovementioned keyboard, mouse, joystick, track ball and control panel. When this operating part 15 is operated, a signal corresponding to the operation content is inputted into the controller 10. Based on this signal, the controller 10 causes the medical image processing apparatus 1 to perform an operation corresponding to the operation content.

### Communication part

The communication part 16 includes a communication interface such as the abovementioned LAN card. Data received by the communication part 16 is inputted into the controller 10 and subjected to a process by the medical image processing apparatus 1. Further, the communication part 16 transmits the data to another apparatus upon receiving a command from the controller 10. The communication part 16 functions as one example of an "accepting part" of the present invention.

### Operation modes

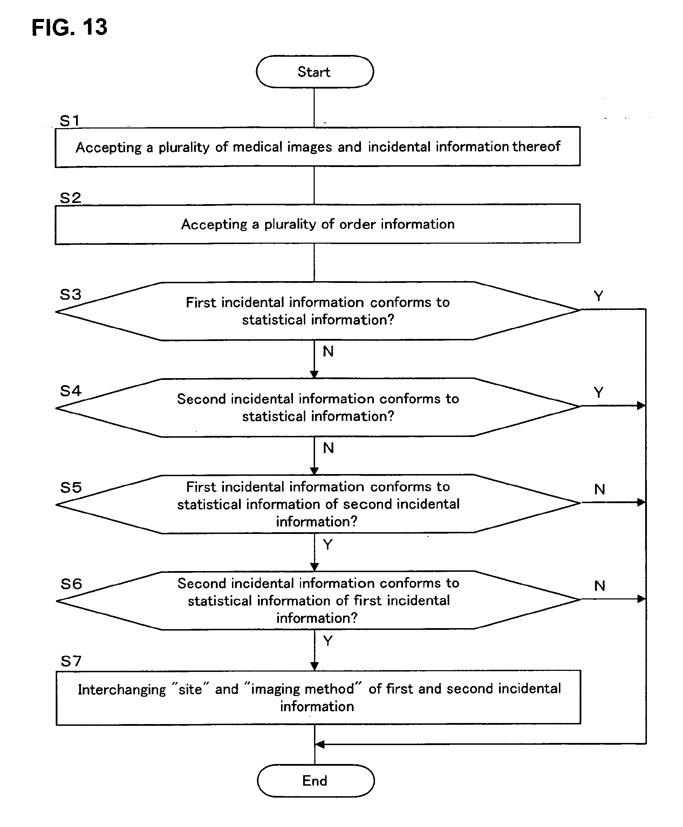

Various operation modes of the medical image processing apparatus 1 having the configuration described above will be explained. The flowcharts shown in Figs. 13 to 21 illustrate one example of an operation mode of the medical image processing apparatus 1.

### First operation mode: Fig. 13

A process of changing incidental information by referring to the imaged-sheet-number statistical information T2 when a plurality of incidental information are inputted into the medical image processing apparatus 1 will be explained referring to the flowchart in Fig. 13.

First, the medical image processing apparatus 1 accepts a plurality of image data of medical images and a plurality of incidental information from the modality 2000 (S1). The controller 10 causes the information storage part 11 to store the accepted image data of the medical images and incidental information (refer to medical images G and incidental information A in Fig. 2).

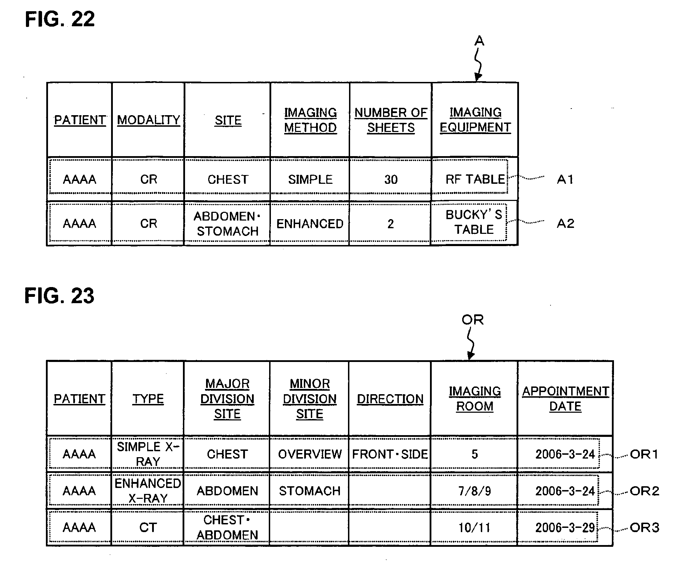

An example of the incidental information is shown in Fig. 22. The incidental information A in Fig. 22 includes first incidental information A1 and second incidental information A2. The first incidental information A1 is incidental to (image data of) a first medical image G1 and the second incidental information A2 is incidental to a second medical image G2. Herein, the medical images G1 and G2 (not shown) are included in the medical image G.

Further, the medical image processing apparatus 1 accepts a plurality of order information from the HIS/RIS 1000 (S2). The plurality of order information include imaging orders of the plurality of medical images accepted at step S1. The controller 10 causes the information storage part 11 to store the accepted order information (refer to order information OR in Fig. 2). The sequence of performing step S1 and S2 is reversible.

An example of the order information is shown in Fig. 23. The order information OR in Fig. 23 includes first order information OR1, second order information OR2, and third order information OR3. The conformity determining portion 121 determines that the first and second order information OR1 and OR2 among the above information correspond to the first and second incidental information A1 and A2, respectively. In other words, the conformity determining portion 121 determines that imaging of the first medical image G1 and generation of the first incidental information have been performed based on the first order information OR1, and imaging of the second medical image G2 and generation of the second incidental information A2 have been performed based on the second order information OR2.

This determining process is executed in the following manner, for example. First, the conformity determining portion 121 reads out the incidental information A, the order information OR and the examination-type statistical information T1 from the information storage part 11. Next, the conformity determining portion 121 recognizes that the examination type corresponding to the modality type "CR" of the first and second incidental information A1 and A2 is "(simple/enhanced) X-ray."

Then, the conformity determining portion 121 excludes the third order information OR3 from the order information corresponding to the first and second incidental information A1 and A2, based on the fact that the examination type of the first and second order information OR1 and OR2 is "(simple/enhanced) X-ray," and the examination type of the third order information OR3 is "CT." This process is based on the fact that the modality type and the examination type are designated as high-accuracy information in the accuracy-designation information 117, as described above.

Furthermore, the conformity determining portion 121 determines that the first order information OR1 and the first incidental information A1 are associated with each other and the second order information OR2 and the second incidental information A2 are associated with each other, based on the sequence of the first and second order information OR1 and OR2 and the sequence of the first and second incidental information A1 and A2. This is the end of the explanation of determination of the association between the order information OR and the incidental information A.

After the order information OR and the incidental information A are associated with each other, the conformity determining portion 121 reads out the imaged-sheet-number statistical information T2 from the information storage part 11.

The conformity determining portion 121 determines whether the first incidental information A1 conforms to the imaged-sheet-number statistical information T2 (S3). This determining process is executed in the following manner, for example.

First, the conformity determining portion 121 obtains the content "CR" of the "modality" section, the content "chest" of the "site" section, the content "simple" of the "imaging method" section, and the content "30" of the "number of sheets" section, from the first incidental information A1. In the same way, the conformity determining portion 121 obtains the content "CR" of the "modality" section, the content "abdomen and stomach" of the "site" section, the content "enhanced" of the "imaging method" section, and the content "2" of the "number of sheets" section, from the second incidental information A2. Among the information, the content of the "number of sheets" section (imaged-sheet-number) is high-accuracy information.

The conformity determining portion 121 refers to the examination-type statistical information T1, thereby recognizing that the modality type "CR" and imaging method "simple" of the first incidental information A1 are associated with the examination type "simple X-ray". In the same way, the conformity determining portion 121 recognizes that the modality type "CR" and imaging method "enhanced" of the second incidental information A2 are associated with the examination type "enhanced X-ray."

Next, the conformity determining portion 121 specifies, from the imaged-sheet-number statistical information T2, statistical information corresponding to the examination type "simple X-ray" and the content "chest" of the "site" section of the first incidental information A1. In this example, statistical information corresponding to the examination type "X-ray" and the major division site "chest" (i.e. statistical information in the first and second columns of the imaged-sheet-number statistical information T2) are specified.

In the same way, the conformity determining portion 121 specifies, from the imaged-sheet-number statistical information T2, statistical information corresponding to the examination type "enhanced X-ray" and the content "abdomen, stomach" of the "site" section of the second incidental information A2. In this example, statistical information corresponding to the examination type "X-ray," the major division site "abdomen," and the minor division site "stomach" (i.e. statistical information in the fourth column of the imaged-sheet-number statistical information T2) are specified.

Subsequently, the conformity determining portion 121 determines whether the number of imaged sheets "30" in the first incidental information A1 conforms to the specified statistical information. This process will now be concretely explained. The statistical information in the first column of the imaged-sheet-number statistical information T2 provides the average value "2.3" of the number of imaged sheets, the standard deviation "0.5," the minimum value "2" and the maximum value "4." Further, the statistical information in the second column provides the average value "5.0" of the number of imaged sheets, the standard deviation "1.3," the minimum value "3" and the maximum value "10."

Compared with this, the number of imaged sheets in the first incidental information A1 is "30," which is much greater than both the maximum values. Further, when the probability that the number of imaged sheets becomes "30" is calculated based on both the average values and both the standard deviations, this probability is extremely small, which is equal to or less than the abovementioned predetermined value. From the above, the conformity determining portion 121 determines the number of imaged sheets "30" to be "nonconforming" (S3; N). When it is determined to be "conforming" (S3; Y), the process of the operation mode ends. This is the end of the explanation of step S3.

When the first incidental information A1 is determined to be "nonconforming" (S3; N), the conformity determining portion 121 determines whether the second incidental information A2 conforms to the statistical information in the fourth column (X-ray, abdomen, stomach) of the imaged-sheet-number statistical information T2 (S4) in the same manner as in step S3. The conformity determining portion 121 determines it to be "nonconforming" based on the content of the second incidental information A2 shown in Fig. 22 and the statistical information in the fourth column shown in Fig. 4 (S4; N). When it is determined to be "conforming" (S4; Y), the process of the operation mode ends.

When both the first and second incidental information A1 and A2 are determined to be "nonconforming" (S4; N), the conformity determining portion 121 determines whether the first incidental information A1 conforms to the statistical information in the first and second columns of the imaged-sheet-number statistical information T2 (S5), in the same manner as in step S3. In this example, it is determined to be "conforming" (S5; Y). When it is determined to be "nonconforming" (S5; N), the process of the operation mode ends.

Furthermore, the conformity determining portion 121 determines whether the second incidental information A2 conforms to the statistical information in the fourth column of the imaged-sheet-number statistical information T2 (S6), in the same manner as in step S3. In this example, it is determined to be "conforming" (S6; Y). When it is determined to be "nonconforming" (S6; N), the process of the operation mode ends.

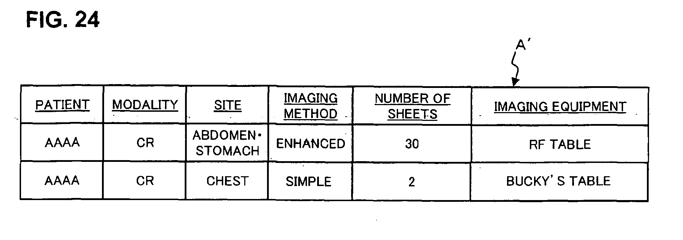

From the above, it appears that the "site" and "imaging method" sections of the first and second incidental information A1 and A2 are have been mixed up and inputted. The information changing part 12 interchanges the contents of the "site" sections, and interchanges the contents of the "imaging method" sections, of the first and second incidental information A1 and A2 (S7). The result of this interchange is shown in Fig. 24. The incidental information A' shown in Fig. 24 is the incidental information A1 and A2 in Fig. 22 in which the "site" sections and the "imaging method" sections are interchanged, respectively.

Herein, in a case where information of a lower level than the interchanged information is included in the first and second incidental information A1 and A2 in the hierarchical structure indicated in the data hierarchy information 113, the information changing part 12 also interchanges the lower-level information (this applies to operation modes below).

Since the interchanged information is the "major division site" in this example, the information changing part 12 interchanges all the information in the first and second incidental information A1 and A2, for information of a lower level than the "major division site" in the order hierarchy information R1 (except for those already interchanged). As a result, for example, the contents of the "imaging equipment" sections are also interchanged (because the "imaging equipment" and the "imaging room" are previously associated with each other).

This is the end of the first operation mode of the medical image processing apparatus 1. The incidental information A having been corrected in this way is transmitted, incidental to the image data G of a medical image, to the server 4000 and the archive apparatus 3000, and provided to the electronic medical record terminal 5000 and the interpretation terminal 6000 (this also applies to the operation modes below).

The changed contents in the incidental information according to the first operation mode are transmitted to the information updating part 13, whereby the imaged-sheet-number statistical information T2 is updated (this also applies to the operation modes below).

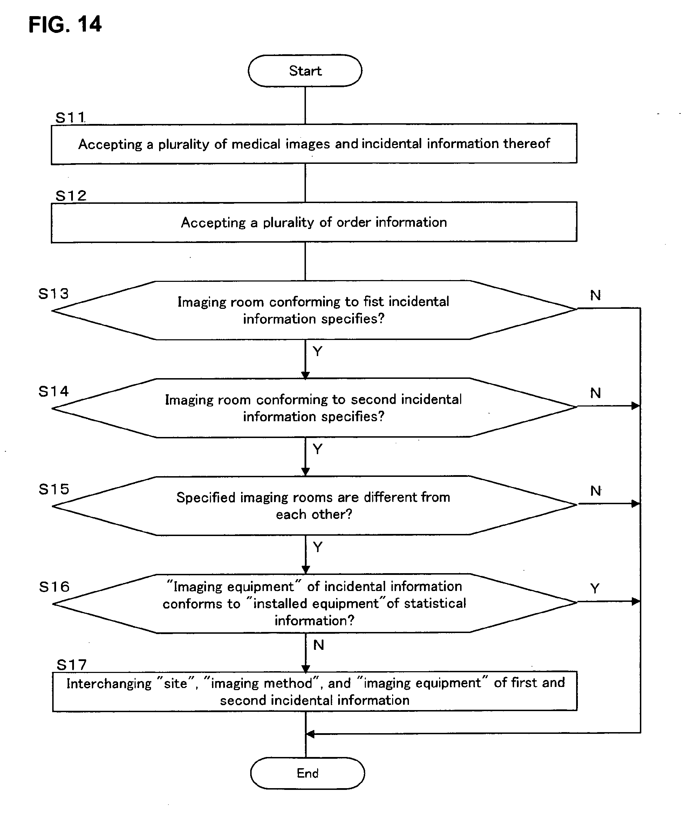

### Second operation mode: Fig. 14

Next, a process of changing incidental information by referring to the imaging-room statistical information T3 when a plurality of incidental information are inputted into the medical image processing apparatus 1 will be explained referring to a flowchart of Fig. 14.

First, the medical image processing apparatus 1 accepts a plurality of image data of medical images and a plurality of incidental information from the modality 2000 (S11), and stores them into the information storage part 11. The incidental information shall be the incidental information A of Fig. 22.

Further, the medical image processing apparatus 1 accepts a plurality of order information from the HIS/RIS 1000 (S12), and stores them into the information storage part 11. The order information shall be the order information OR of Fig. 23.

The conformity determining portion 121 reads out the incidental information A, the order information OR and the examination-type statistical information T1 from the information storage part 11, associates the first order information OR1 with the first incidental information A1, and also associates the second order information OR2 with the second incidental information A2, in the same manner as in the first operation mode.

After associating the order information OR with the incidental information A, the conformity determining portion 121 reads out the imaging-room statistical information T3 from the information storage part 11.

The conformity determining portion 121 specifies an imaging room conforming to the fist incidental information A1 from among imaging rooms shown in the imaging-room statistical information T3 (S13). This specification process is executed in the following manner, for example.

First, the conformity determining portion 121 specifies (identification information of) an imaging room corresponding to the contents of the "modality" section, the "site" section and the "imaging method" section of the incidental information A1. In this example, the contents of these sections are "CR" (= "X-ray"; from the examination-type statistical information T1), "chest," and "simple," so that the imaging room "5" in which the "installed equipment" section is "Bucky's table X-ray" and the "examination and site" section is "chest: simple/enhanced," and the imaging room "7" in which the "installed equipment" section is "RF table X-ray" and the "examination and site" section is "chest: simple/enhanced," are specified. Herein, the contents of the "imaging equipment" sections in the first and second incidental information and the contents of the "installed equipment" in the imaging-room statistical information T3 are reversed (such mistakes are corrected by the following process).

Next, the conformity determining portion 121 executes the aforementioned process of determining conformity/nonconformity based on the use rates of the specified two imaging rooms "5" and "7," which are 73% and 13%, respectively, thereby determining that the imaging room "7" is nonconforming and the imaging room "5" is conforming. As a result, the imaging room "5" is specified as an imaging room conforming to the first incidental information A1 (S13; Y). In a case where no conforming imaging room is specified (S13; N), the process of this second operation mode ends.

Further, the conformity determining portion 121 specifies an imaging room conforming to the second incidental information A2 from among the imaging rooms shown in the imaging-room statistical information T3 (S14). In this example, the imaging room "7" is specified based on the contents of the "modality" section, the "site" section and the "imaging method" section of the incidental information A1, which are "CR," " abdomen, stomach," and "enhanced" (S14; Y), in the same manner as in step S13. In a case where no conforming imaging room is specified (S14; N), the process of this second operation mode ends.

Next, the conformity determining portion 121 determines whether the imaging rooms specified for the respective first and second incidental information A1 and A2 are different from each other (S15). When the specified imaging rooms are identical (S15; N), the process of this second operation mode ends.

On the other hand, when the specified imaging rooms are different (S15; Y), the conformity determining portion 121 determines whether the contents of the "imaging equipment" sections of the first and second incidental information A1 and A2 conform to the content of the "installed equipment" section of the imaging-room statistical information T3 (S16). When it is determined "conforming" (S16; Y), the process of this second operation mode ends.

When it is determined "nonconforming" (S16; N), the information changing part 12 interchanges the contents of the "site" sections, the "imaging method" sections and the "imaging equipment" sections of the first and second incidental information A1 and A2 (S17). This is the end of the second operation mode of the medical image processing apparatus 1.

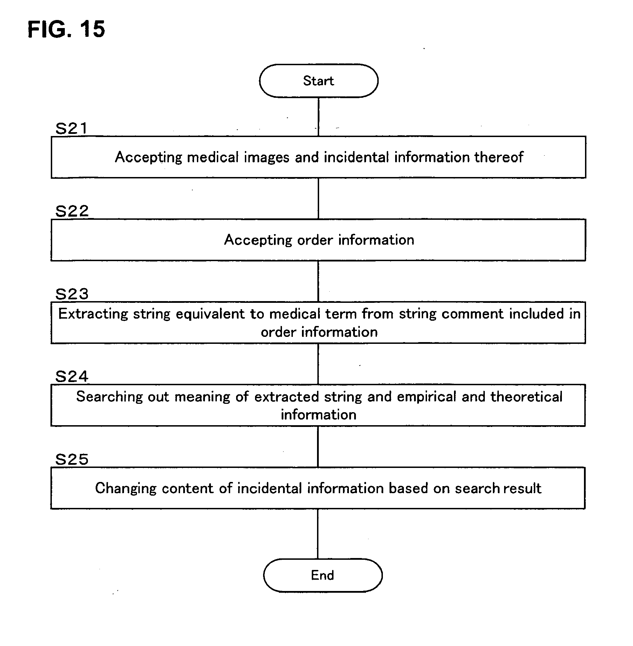

### Third operation mode: Fig. 15

Next, a process of changing incidental information based on a string comment included in order information will be explained referring to a flowchart of Fig. 15.

First, the medical image processing apparatus 1 accepts image data of a medical image and incidental information from the modality 2000 (S21), and stores them into the information storage part 11.

Further, the medical image processing apparatus 1 accepts order information from the HIS/RIS 1000 (S22), and stores them into the information storage part 11. This order information shall include a string comment.

Next, the string-information extracting portion 123 extracts a string equivalent to a medical term stored on the medical-knowledge database 111, from the string comment included in the order information (S23).

Subsequently, the medical-term search portion 124 searches out the meaning of the string extracted from the string comment and empirical and theoretical information, from the medical-knowledge database 111 (S24). For example, the medical-term search portion 124 obtains the fact that the number of imaged sheets will be approximately several tens based on the string comment such as "focused on imaging of the abdomen."

The information changing part 12 changes the content of incidental information based on the meaning of the string and empirical and theoretical information having been searched (S25). For example, when it is obtained that the number of imaged sheets (high-accuracy information; refer to the accuracy-designation information 117) of the second incidental information A2 shown in Fig. 22 will be approximately several tens, it is possible to interchange the low-accuracy information (such as "site" and "imaging method") of the first and second incidental information A1 and A2.

Further, it is possible to configure the information changing part 12 so as to, in such a case that certain low-accuracy information is uniquely determined when certain high-accuracy information has been determined according to empirical and theoretical information, change low-accuracy information in incidental information to this unique content. This is the end of explanation of the third operation mode.

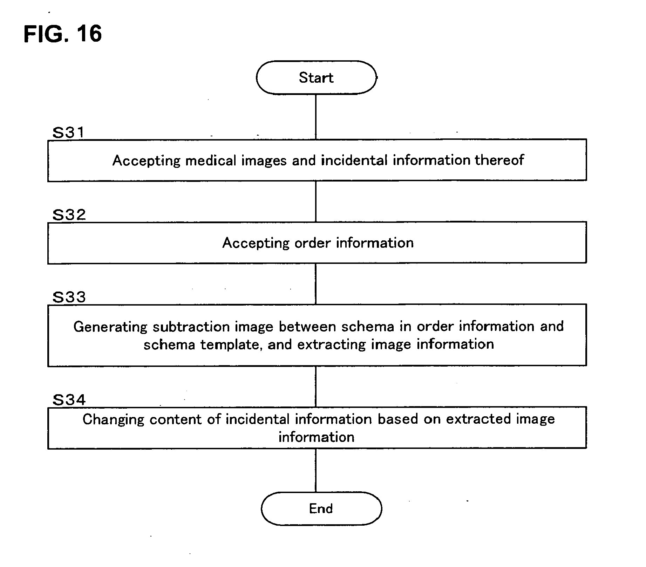

### Fourth operation mode: Fig. 16

Next, a process of changing incidental information based on image comments included in order information will be explained referring to the flowchart in Fig. 16.

First, the medical image processing apparatus 1 accepts image data of a medical image and incidental information from the modality 2000 (S31), and stores them into the information storage part 11.

In addition, the medical image processing apparatus 1 accepts order information from the HIS/RIS 1000 (S32), and stores them into the information storage part 11. This order information shall include image comments (schema).

Next, the comment-image analysis portion 125 reads out the schema template 114 (corresponding to the schema in the order information) from the information storage part 11. Then, the comment-image analysis portion 125 generates a subtraction image between the schema in the order information and the schema template 114, and then extracts the image information written into the schema (S33).

A specific example of the process at step S33 will be explained. In a case where the schema of the order information is the schema C1 in Fig. 10 (schema of the lung region), the comment-image analysis portion 125 reads out the lung-region schema template 114a and calculates a subtraction image between the schema C1 and the lung-region schema template 114a, thereby extracting the image information c1 in Fig. 10.

Then, the information changing part 12 changes the contents of the incidental information based on the image information extracted from the schema (S34).

A specific example of the process at step S34 will be explained. In a case where image information such as a graphic has been extracted as shown in Fig. 10, for example, the position of the extracted image information in the schema (schema template 114) is specified. It is possible to easily execute this process by employing, for example, the alignment result between the schema and the schema template 114 at the time of generation of a subtraction image.

The information changing part 12 can recognize, based on the position of the specified image information, what site of a human body has been the focus of an imaged medical image. As an example, the schema C1 in Fig. 10 will be explained. The information changing part 12 recognizes, from the fact that the image information c1 of the schema C1 is written into the bottom region on the outer side of the left lung (attention region), that the medical image has been imaged focusing on this attention region.

The information changing part 12 can correct the contents of the "site" section in the incidental information based on the position of this attention region. In addition, the information changing part 12 can search out information associated with the position of this attention region from the medical-knowledge database 111, thereby correcting the contents of the "imaging method" section in the incidental information based on the searched information.

Meanwhile, when image information consisting of a string has been extracted, the comment-image analysis portion 125 searches out the meaning and the like of this string from the medical-knowledge database 111, as described above. The information changing part 12 can correct the contents of the incidental information based on the search results.

### Fifth operation mode

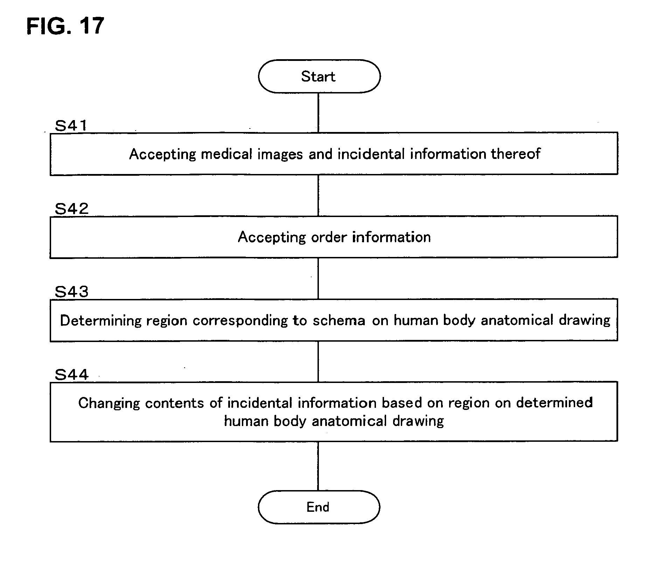

Furthermore, a process of changing incidental information based on image comments included in order information will be explained referring to the flowchart in Fig. 17.

First, the medical image processing apparatus 1 accepts image data of a medical image and incidental information from the modality 2000 (S41), and stores them into the information storage part 11.

In addition, the medical image processing apparatus 1 accepts order information from the HIS/RIS 1000 (S42), and stores them into the information storage part 11. This order information shall include image comments (schema).

Next, the schema-region determining portion 126 reads out the human-body-atlas information 115 from the information storage part 11, and determines a region corresponding to the schema on the human body anatomical drawing shown in this human-body-atlas information 115 (S43).

Then, the information changing part 12 changes the contents of the incidental information based on the region of the schema on this human body anatomical drawing (S44).

A specific example of the process at step S44 will be explained. Human-body-site identification information, such as the name of a human body site corresponding to each of positions of the human body anatomical drawing in the human-body-atlas information 115, is previously associated with each of the positions. For example, a human-body site "lung" is associated with a position equivalent to the lung on the human body anatomical drawing. The information changing part 12 obtains the human-body-site identification information equivalent to the region determined at step S43, and changes the contents of the "site" section, the contents of the "imaging method" section and so on in the incidental information, based on the human body site specified by this identification information.

### Sixth operation mode

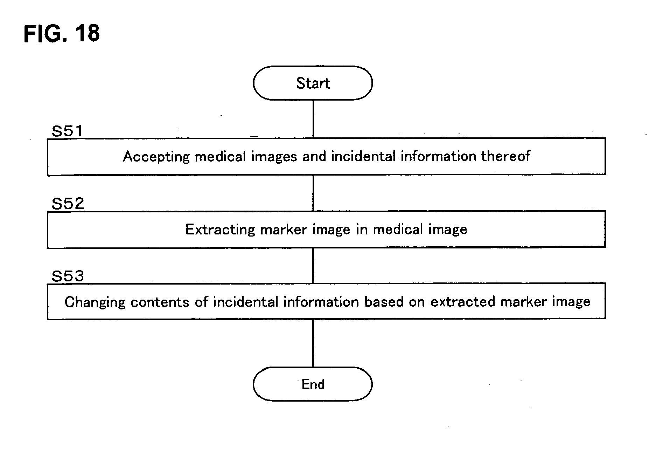

A process of changing incidental information based on a marker image in a medical image will be explained referring to the flowchart in Fig. 18.

First, the medical image processing apparatus 1 accepts image data of a medical image and incidental information from the modality 2000 (S51), and stores them into the information storage part 11. Marker images such as L-R marks or various markers shall be embedded in this medical image.

The marker-extracting portion 128 reads out L-R-mark-pattern information or marker-pattern information in the image-analysis information 116, from the information storage part 11. Then, the marker-extracting portion 128 analyzes image data of this medical image based on the L-R-mark-pattern information or the marker-pattern information, thereby extracting a marker image in a medical image (S52).

The information changing part 12 changes the contents of the incidental information based on the extracted marker image (S53). A specific example of the process at step S53 will be explained. When the marker image embedded in the medical image is the L-R mark, letters "L" and "R" are extracted at step S52. The information changing part 12 specifies the horizontal direction of that medical image by applying, for instance, a known character-recognition technique or the like to the extracted letter. Then, the information changing part 12 corrects the contents of the "site" section and the "imaging method" section and so on in the incidental information based on the specified horizontal direction.

In addition, when the marker image embedded in the medical image is a marker indicating a reference position or an attention position, it is possible to specify the position of the marker in that medical image based on the extraction result at step S52. Moreover, when the marker has a predetermined meaning like a character (string) or a graphic, it is possible to specify the meaning thereof by employing a known character-recognition technique or pattern-recognition technique. Then, it is possible to correct the contents of the "site" section or the "imaging method" section and so on in the incidental information based on the specified meaning.

### Seventh operation mode

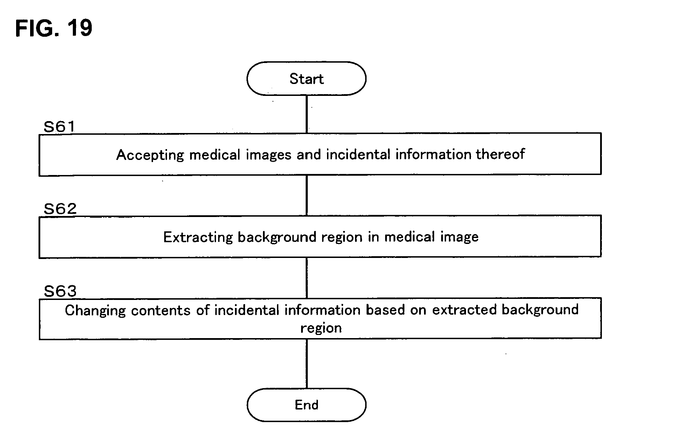

A process of changing incidental information based on a background region of a medical image will be explained referring to the flowchart in Fig. 19.

First, the medical image processing apparatus 1 accepts image data of a medical image and incidental information from the modality 2000 (S61), and stores them into the information storage part 11. The background-region extracting portion 129 reads out threshold information of pixel values in the image-analysis information 116, from the information storage part 11. Then, the background-region extracting portion 129 analyzes image data of the medical image based on this threshold information of the pixel values, thereby extracting a background region in the medical image (S62).

The information changing part 12 changes the contents of the incidental information based on the extracted background region (S63). A specific example of the process at step S63 will be explained. A background region of a medical image has a characteristic pattern that reflects the classification of the medical image. The information changing part 12 can specify the classification of the medical image based on the pattern of the extracted background region and correct the contents of the "site" section or the "imaging method" section and so on in the incidental information based on the specification result.

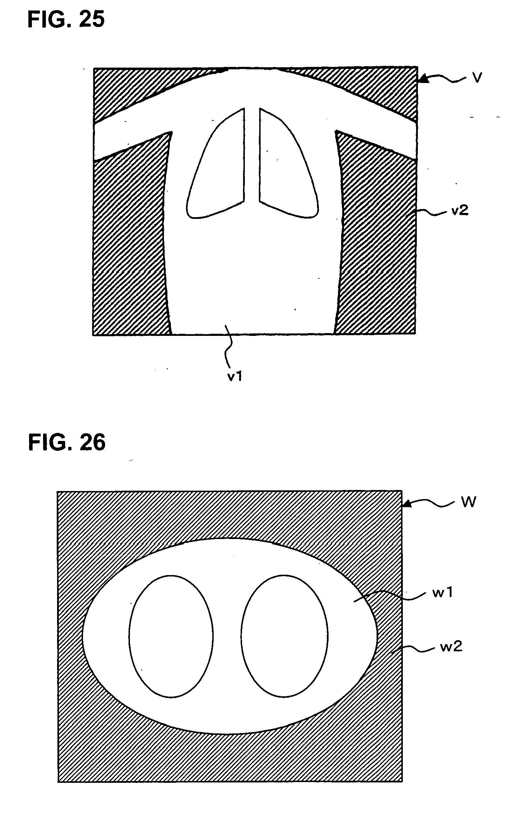

Examples of background regions corresponding to classifications (imaging-types) of medical images are shown in Figs. 25 to 28. A medical image V shown in Fig. 25 is an image of the upper body (chest overview) of a subject taken from the front by using an X-ray-diagnostic apparatus (CR, DR). This medical image V includes a subject image region v1 equivalent to a detection region of an X-ray having transmitted through the subject, and a background region v2 equivalent to a detection region of an X-ray having not transmitted through the subject. The background region v2 of the medical image V has four discrete connected components.

When the number of the connected components in the background region extracted at step S62 is four (it may be add to the criteria that each connected component is positioned on the corner of the image), the information changing part 12 can specify that the examination type (modality type) of this medical image is "X-ray," the imaged site is "chest, overview," the imaging direction is "front side" and so on, thereby correcting the incidental information based on the specification result.

A medical image W shown in Fig. 26 is a tomographic image (axial image) of the chest (lung) of a subject having been imaged by using an X-ray CT apparatus (or an MRI apparatus). This medical image W includes the subject image region w1 and the background region w2. The background region w2 has a shape composed of a single connected component, excluding the subject image region w1 in the middle of the image.

When the background region extracted at step S62 has a shape composed of a single connected component excluding the middle of the image, the information changing part 12 can specify that the examination type (modality type) of this medical image is "CT" or "MR." Incidentally, the distinction between "CT" and "MR" can be made by, for example, applying a process of calculating the image quality of an image by analyzing pixel values.

In addition, in consideration that an image region with an almost symmetrical shape (i.e. an image region equivalent to both the lungs) exists in the subject image region w1, the information changing part 12 can specify that the imaged site is "chest, overview," the imaging direction (cross-sectional direction) is an "axial image," and so on. Then, the information changing part 12 can correct the incidental information based on this specification result.

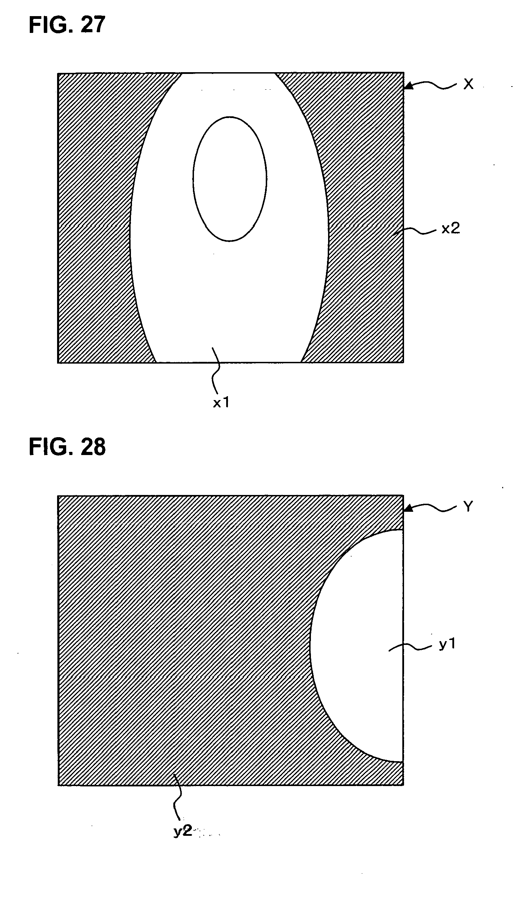

A medical image X shown in Fig. 27 is an image of the upper body (chest overview) of a subject taken from the side by using an X-ray diagnostic apparatus. This medical image X includes a subject-image region x1 and a background region x2. The background region x2 has two discrete connected components separated in the horizontal direction on the image.

When the background region extracted at step S62 has two discrete connected components separated in the horizontal direction on the image, the information changing part 12 can specify that the examination type (modality type) of this medical image is "X-ray," the imaged site is "chest, overview," the imaging direction is "side surface" and so on, thereby correcting the incidental information based on this specification result.

A medical image Y shown in Fig. 28 is an image of the right breast of a subject taken (from the side) by using an X-ray diagnostic apparatus (mammography). This medical image Y includes a subject image region y1 and a background region y2. The background region y2 has a shape composed of a single connected component excluding the subject image region y1 positioned on the right end of the image. Incidentally, the background region of the medical image of the left breast has a shape composed of a single connected component excluding the subject image region positioned on the left end of the image.

When the background region extracted at step S62 has a shape composed of a single connected component excluding the right end of the image, the information changing part 12 can specify that the examination type (modality type) of this medical image is "X-ray" or "mammography," the imaged site is "right breast," the imaging direction is "side surface (right direction)" and so on, thereby correcting the incidental information based on this specification result.

### Eighth operation mode

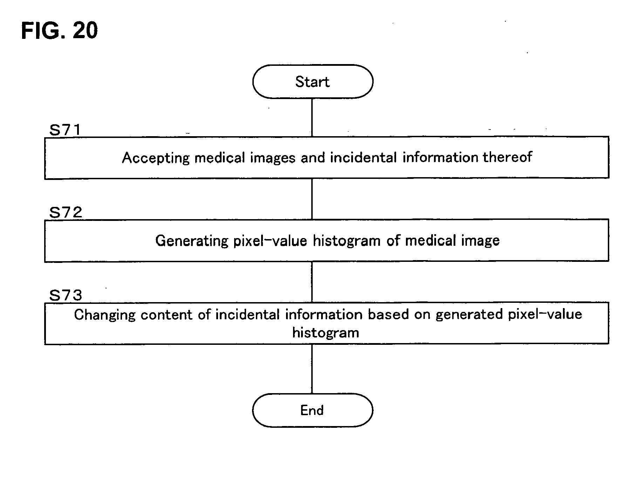

A process of changing incidental information based on a background region of a medical image will be explained referring to a flowchart of Fig. 20.

First, the medical image processing apparatus 1 accepts image data of a medical image and incidental information from the modality 2000 (S71), and stores them into the information storage part 11.

The histogram generating portion 130 reads out histogram-associated information in the image-analysis information 116, from the information storage part 11. Then, the histogram generating portion 130 generates a pixel-value histogram of the medical image (S72).

The information changing part 12 changes the content of the incidental information based on the generated pixel-value histogram and the histogram-associated information (S73).

The process at step S73 will be explained in more detail. As described above, the pixel-value histogram has a characteristic pattern reflecting the imaged site and imaging direction of the medical image. Further, the histogram-associated information defines association between the pattern of the pixel-value histogram and the imaged site and imaging direction.

The information changing part 12 selects an imaged site and imaging direction conforming to the pattern of the pixel-value histogram generated at step S72, from the histogram-associated information. Then, the information changing part 12 changes the content of the incidental information based on the information on the selected imaged site and imaging direction.

### Ninth operation mode

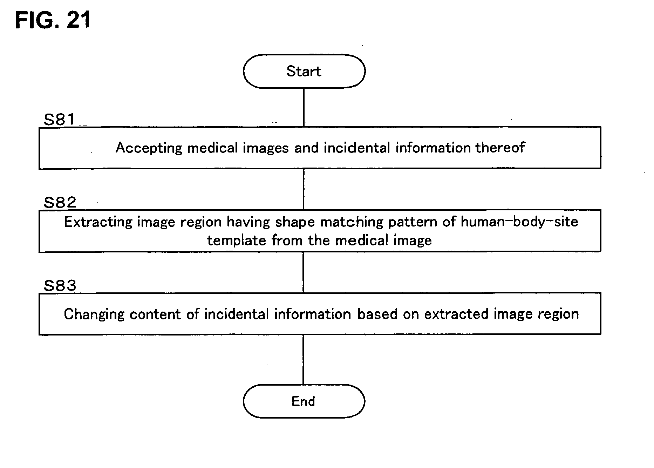

A process of changing incidental information based on a pattern of a human body site included in a medical image will be explained referring to a flowchart of Fig. 21.

First, the medical image processing apparatus 1 accepts image data of a medical image and incidental information from the modality 2000 (S81), and stores them into the information storage part 11.

The pattern extracting portion 131 reads out a human-body-site template in the image-analysis information 116, from the information storage part 11. Then, the pattern extracting portion 131 extracts an image region having a shape matching the pattern of the human body site indicated in the human-body-site template, from the medical image (S82).

The information changing part 12 changes the content of the incidental information based on the extracted image region (S83). The process at step S83 will be explained in more detail. The information changing part 12 specifies the imaged site and imaging direction of the medical image, based on the human body site indicated by the human-body-site template corresponding to the extracted image region, and changes the content of the incidental information based on the specified information.

This is the end of the operation modes of the medical image processing apparatus 1. For the medical image processing apparatus according to the present invention, it is sufficient to perform at least one operation mode among the aforementioned first to ninth operation modes. In this case, for the apparatus, it is sufficient to comprise only component parts necessary for the performed operation mode. For example, a medical image processing apparatus capable of performing only the first, third and sixth operation modes comprises the conformity determining portion 121, the comment-information analysis portion 122 including the string-information extracting portion 123 and the medical-term search portion 124, and the medical-image analysis portion 127 including marker-extracting portion 128.

### Actions and Advantages

Actions and advantages of the medical image processing apparatus 1 according to the present embodiment will be explained.

The medical image processing apparatus 1 acts so as to accept image data of a medical image and incidental information thereof from the modality 2000 and the like and compare this incidental information with predetermined reference information to change the incidental information.

Herein, the predetermined reference information is the medical-knowledge database 111, the statistical-information database 112, the data hierarchy information 113, the schema template 114, the human-body-atlas information 115, the image-analysis information 116, the accuracy-designation information 117, and so on.

Furthermore, the predetermined reference information is a marker image, background region and pixel-value histogram obtained by analyzing image data of a medical image, various kinds of information specified therefrom, and so on.

Thus, according to the medical image processing apparatus 1, it is possible to automatically correct incidental information of image data of a medical image by referring to the predetermined reference information.

In particular, as explained in the first and second operation modes, according to the medical image processing apparatus 1, in a case where the contents of a plurality of incidental information have been switched, it is possible to specify and interchange the switched contents, thereby correcting.

As a result, even when the contents of a plurality of order information are mixed up and a plurality of incidental information thereof are created due to human error or operational error of the modality 2000, it is possible to automatically correct the contents of the plurality of incidental information.

This correction process is to correct information with a high possibility of mistakes (low-accuracy information) based on information with a low possibility of mistakes (high-accuracy information), among various types of information included in each of the plurality of incidental information. At this moment, mistakes in low-accuracy information are detected by using the statistical relationship between the high-accuracy information and the low-accuracy information (statistical-information database 112). Therefore, it is possible to detect and correct mistakes in low-accuracy information with high accuracy.

The contents of order information are likely to be mixed up particularly when imaging is repeated. Further, when continuous imaging is performed for a plurality of patients, an operator may input information of the previous or next patient due to an operational error. Furthermore, when a plurality of examinations are performed at one time, one order information includes a plurality of suborders (refer to Fig. 23). In this case, the contents of the suborders may be mixed up and inputted. The first and second operation modes are to correct creation mistakes in incidental information caused in this way.

Further, as explained in the third to fifth operation modes, the medical image processing apparatus 1 acts so as to correct incidental information by referring to comment information included in order information. In general, the comment information includes important information such as notanda in imaging based on the order information. Therefore, it becomes possible to detect and correct mistakes in the incidental information with high accuracy.

Further, as explained in the sixth to ninth operation modes, the medical image processing apparatus 1 acts so as to analyze image data of a medical image accompanied by incidental information to obtain predetermined reference information, and correct the incidental information by using the reference information. Therefore, even when the contents of the incidental information do not conform to the medical image, it is possible to detect and correct mistakes in the incidental information.

The actions and advantages of the medical image processing apparatus 1 as described above make it possible to prevent incidents (troubles that may potentially occur, and the like) or accidents (troubles having occurred actually), such as mix-up of patient information or order information.

Further, a configuration of correcting the contents of incidental information by utilizing the abovementioned predetermined reference information is applied, so that it is possible to standardize the contents of the information used in a medical institution. Consequently, it is possible to effectively and accurately perform case search, course tracking, statistical case measure improvement, and so on.

Further, it is also possible to perform interpretation of a medical image, access to an electronic medical record, and so on, more efficiently by standardizing the contents of the information used in a medical institution. For example, when performing comparative interpretations of a medical image, it is possible to input, as a standardized search keyword, information on an imaged site, imaging method and so on, and to search and display an image according to it. Furthermore, for imaging by using a contrast agent or the like, it is possible to search and display images with the same phase by having standardized a term indicating a certain phase of imaging.

Further, when incidental information has been corrected, the medical image processing apparatus 1 acts so as to update the contents stored in the information storage part 11 based on this correction result. Specifically, it acts so as to update the statistical-information database 112 in response to the fact that low-accuracy information among incidental information has been corrected. That makes it possible to improve the accuracy in the process of correcting incidental information through time (in other words, as the apparatus continues to be operated, the correction accuracy improves).

Particularly, it is possible to build up the specific statistical-information database 112 in accordance with specific conditions for each medical institution (e.g. conditions such as the type or number of an installed modality and the type or frequency of an examination (imaging) to be performed. Therefore, it is possible to improve the accuracy in correction of incidental information by utilizing the statistical-information database 112 that reflects conditions for each medical institution.

Incidentally, a process of updating the stored contents in the information storage part 11 can be performed as necessary at any timing other than when incidental information has been corrected. For example, it is possible to sequentially update the medical-knowledge database 111, thereby building up a database that sequentially reflects medical knowledge progressing day by day. As a result, it is possible to improve the accuracy in correction of incidental information or the like. In addition, it is also possible to verify the contents of the medical-knowledge database 111 to review the system.

Further, when certain information in incidental information has been corrected, this medical image processing apparatus 1 acts so as to apply corrections to information defined in levels lower than this information by referring to the data hierarchy information 113. As a result, it is possible to improve the accuracy of the process of correcting incidental information, and accelerate the process.

### SECOND EMBODIMENT

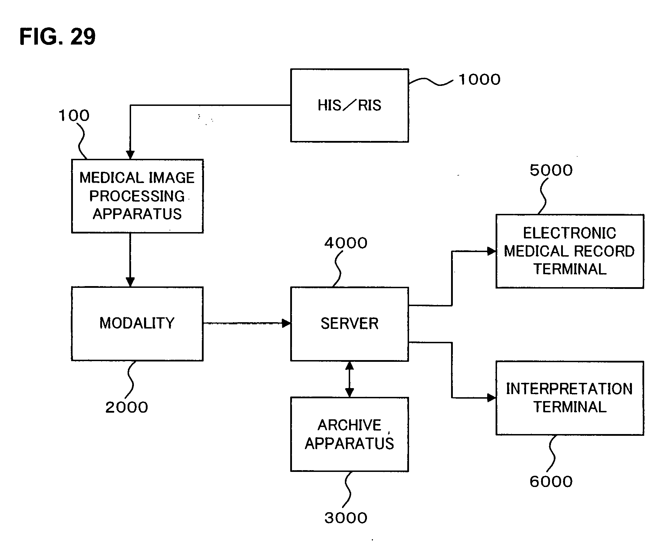

A second embodiment of the medical image processing apparatus according to the present invention will be explained. Fig. 29 illustrates one example of the configuration of a medical system operated by including the medical image processing apparatus according to this embodiment. This medical system comprises a medical image processing apparatus 100, the HIS/RIS 1000, the modality 2000, the archive apparatus 3000, the server 4000, the electronic medical record terminal 5000, and the interpretation terminal 6000, as in the first embodiment. These apparatuses are connected via a communication line such as a LAN (Local Area Network) and a leased line.

The HIS/RIS 1000, the modality 2000, the archive apparatus 3000, the server 4000, the electronic medical record terminal 5000 and the interpretation terminal 6000 each have the same configuration as in the first embodiment.

The medical image processing apparatus 100 accepts order information outputted from the HIS/RIS 1000, and transmits the order information to the modality 2000 after correcting the order information.

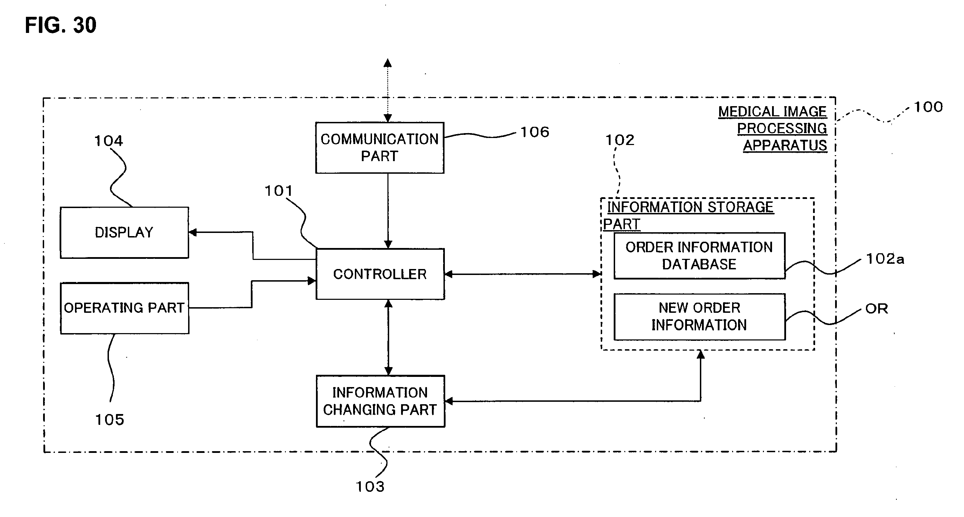

Fig. 30 illustrates one example of the configuration of this medical image processing apparatus 100. The medical image processing apparatus 100 shown in Fig. 30 comprises a controller 101, an information storage part 102, an information changing part 103, a display 104, an operating part 105, and a communication part 106.

The controller 101 controls the operation of each part of the medical image processing apparatus 100. The information storage part 102 functions as one example of the "storing part" of the present invention, and stores an order-information database (order-information DB) 102a. This order-information database 102a is a database formed by accumulating order information inputted from the HIS/RIS 1000 in the medical image processing apparatus 100 in the past.

The order-information database 102a stores order information searchably with various kinds of information forming the order information. The order information can be searched by using, as keywords, information such as patient-identification information (patient name and patient ID), imaged site (major division site, medium division site, and minor division site), an examination type, (identification information of) an imaging room, imaging direction, imaging method, and a manipulation.

The order-information database 102a may include the order hierarchy information R1 (refer to Fig. 6) of the first embodiment. This order hierarchy information R1 illustrates one example of the hierarchical structure of data included in order information. When information of a certain hierarchy is designated, it is possible to specify information that can appear in lower hierarchies by referring to this order hierarchy information R1.

When new order information is inputted from the HIS/RIS 1000 (the new order information OR is stored in the information storage part 102), the information changing part 103 changes the contents of the new order information OR based on the order-information database 102a. A specific example of this change process will be described later. The information changing part 103 functions as one example of the "changing part" of the present invention.

The display 104 has the same configuration as the display 14 of the first embodiment, and functions in the same manner. Further, the operating part 105 has the same configuration as the operating part 15 of the first embodiment, and functions in the same manner. Furthermore, the communication part 106 has the same configuration as the communication part 16 of the first embodiment, and functions in the same manner. The communication part 16 functions as one example of the "accepting part" of the present invention.

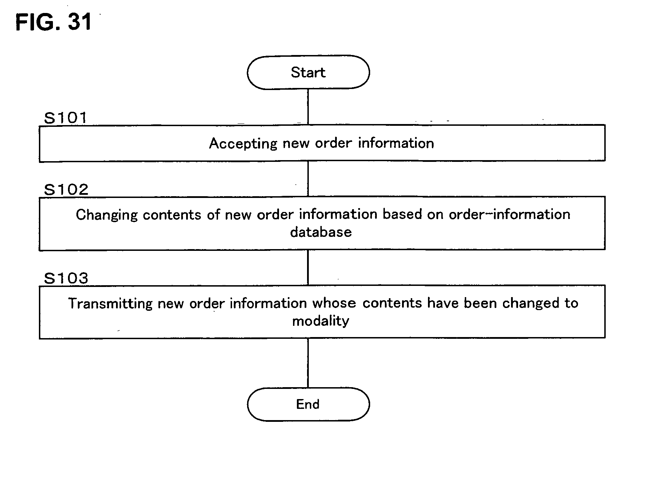

An operation mode of this medical image processing apparatus 100 will be explained. Fig. 31 illustrates one example of the operation mode of the medical image processing apparatus 100.

First, the medical image processing apparatus 100 accepts order information from the HIS/RIS 1000 (S101), and stores as new order information OR in the information storage part 102.

Next, the information changing part 103 reads out the order-information database 102a and the new order information OR from the information storage part 102. Then, the information changing part 103 changes the contents of the new order information OR based on the order-information database 102a (S102).

The controller 101 controls the communication part 106 so as to transmit the new order information OR whose contents have been changed at step S102, to the modality 2000 (S103).

A specific example of the process at step S102 will be explained. As a first specific example, a case where an order for comparative interpretation, such as a course observation, has been issued will be explained. First, the information changing part 103 obtains patient-identification information from the "patient" section of the new order information OR. Next, the information changing part 103 searches the order-information database 102a by using the patient-identification information as a search keyword. As a result, past order information of that patient can be obtained. The information changing part 103 compares the contents of the past order information with the contents of the new order information OR, thereby determining the difference therebetween. In a case where there is different information (besides information in the "reservation date" section), the information changing part 103 changes the different information of the new order information OR to the information of the past order information. As a result, the new order information OR with the same order contents as information recorded in the past can be obtained, whereby it is possible to favorably perform the comparative interpretation.

As a second example, a case of utilizing statistical information of past order information will be explained. As this statistical information, it is possible to use, for example, the imaged-sheet-number statistical information T2 of Fig. 4, the imaging-room statistical information T3 of Fig. 5, and so on. The information changing part 103 compares the new order information OR with this statistical information to execute the same process as in the first embodiment (refer to the first and second operation modes), thereby changing the contents of the new order information OR. As a result, it is possible to change information that does not conform to prior statistics.

When certain information of the new order information OR has been changed, the information changing part 103 can also change information in lower levels than the changed information, based on the order hierarchy information R1.

This medical image processing apparatus 100 may also be provided with an updating part configured to update the contents of the order-information database 102a based on the changed contents of the new order information OR, as in the first embodiment. This updating part functions in the same manner as the information updating part 13 of the first embodiment.

### MODIFIED EXAMPLE

The configurations and operation modes described in detail above are only examples for preferable implementations of the medical image processing apparatus according to the present invention. Therefore, it is possible to apply any modification within the scope of the invention as necessary.

For example, the medical image processing apparatus 1 according to the first embodiment can be configured so as to output medical information changed by the information changing part 12.

As one example thereof, it is possible to cause the display 14 to display the medical information after change. The controller 10 executes the display process. Alternatively, the medical image processing apparatus 1 may be connected to a printer (not shown), thereby printing out print out the medical information after change. Together with the controller 10, the display 14 and the printer are examples of the "output part" of the present invention.

As a result, it becomes possible to check whether medical information has been appropriately changed.

Information outputted by the output part is not limited to the medical information after change. For example, it is possible to output both medical information having been changed and medical information having not been changed.

In this case, it is desirable to output the medical information having been changed (changed medical information) and the medical information having not been changed (unchanged medical information) in different output modes. For example, it is possible to output the changed medical information and the unchanged medical information with different colors. Further, when the changed medical information and the unchanged medical information are string information, they can be outputted in different character styles (such as font, size, and line thickness). Furthermore, it is also possible to output a group of the changed medical information and a group of the unchanged medical information in different positions.

By outputting both the changed and unchanged medical information in this way, it is possible to collectively verify medical information included in incidental information. Moreover, it is possible to comprehend what medical information has been changed and what medical information has not been changed, by differentiating the output modes of the changed medical information and the unchanged medical information.

Further, when outputting a plurality of medical information, the output part is capable of outputting the medical information in the order depending on the degree of accuracy. Herein, the degree of accuracy of respective medical information is obtained by referring to the accuracy-designation information 117. When there are two or more medical information with the same accuracy degree, the arrangement order thereof can be set optionally. With such a configuration, it is possible to grasp the accuracy of medical information to be outputted. Furthermore, when outputting medical information in the order of the accuracy thereof, it is possible to output respective medical information in an output mode (such as color and character style) according to the accuracy. As a result, it becomes possible to easily comprehend the accuracy of the medical information to be outputted.

Alternatively, the medical information having been outputted may be manually changed by the output part. For example, in the case of displaying medical information on the display 14, it may configure so that the medical information can be changed employing the operating part 15. At this moment, the operating part 15 is employed as one example of the "operating part" of the present invention.

According this configuration, a doctor or the like can check whether automatically changed medical information is appropriate or not and, if it is not appropriate, manually add corrections. This configuration is considered to be effective, for example, at the time of updating a system or in case of a system failure.

Furthermore, it is possible to configure so as to, when the medical information has been changed with the operating part, update the contents of the associated information stored in the information storage part 11 (for example, the statistical-information database 112), based on the medical information after change. The information updating part 13 executes this process in the same manner as in the first embodiment. According to this configuration, it is possible to improve the accuracy in correction of incidental information.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. A medical image processing apparatus comprising:
an accepting part configured to accept image data of a medical image and incidental information thereof;
a determining part configured to compare predetermined reference information that includes associated information associating a plurality of medical information based on a degree of accuracy, with medical information included in the incidental information, and determine whether the medical information conforms to the predetermined reference information; and
a changing part configured to change the medial information included in the incidental information based on the predetermined reference information when it is determined the medical information does not conform.

2. A medical image processing apparatus according to Claim 1,
wherein:
the associated information is information that associates high-accuracy information with low-accuracy information among the plurality of medical information; and
the changing part changes the low-accuracy information.

3. A medical image processing apparatus according to Claim 2,
wherein:
the accepting part accepts a plurality of image data of medical images and a plurality of incidental information thereof;
the associated information is information that associates imaging-type information indicating an imaging type of the medical image as the low-accuracy information, with statistical information indicating a number of imaged sheets of the medical image as the high-accuracy information;
the determining part determines, for each of the plurality of incidental information, whether association between the imaging-type information and the imaged-sheet-number information conforms to the associated information; and
when two or more of the incidental information are determined not to conform, respectively, the changing part executes a process of interchanging the imaging-type information of the two or more incidental information so as to conform to the associated information, as a process of changing the low-accuracy information.

4. A medical image processing apparatus according to Claim 2,
wherein:
the accepting part accepts a plurality of image data of medial images and a plurality of incidental information thereof;
the associated information is information that associates imaged-site information indicating an imaged site of the medical image as the low-accuracy information, with statistical information indicating a number of imaged sheets of the medical image as the high-accuracy information;
the determining part determines, for each of the plurality of incidental information, whether association between the imaged-site information and the imaged-sheet-number information conforms to the associated information; and
when two or more of the incidental information are determined not to conform, respectively, the changing part executes a process of interchanging the imaged-site information of the two or more incidental information so as to conform to the associated information, as a process of changing the low-accuracy information.

5. A medical image processing apparatus according to Claim 2,
wherein:
the accepting part accepts a plurality of image data of medial images and a plurality of incidental information thereof;
the associated information is information that associates imaging-type information indicating an imaging type of the medical image as the low-accuracy information, with statistical information indicating a frequency of use of an imaging room for capturing the medical image as the high-accuracy information;
the determining part determines, for each of the plurality of incidental information, whether combination of the imaging-type information and the imaging room conforms to the associated information; and
when two or more of the incidental information are determined not to conform, respectively, the changing part executes a process of interchanging the imaging-type information of the two or more incidental information so as to conform to the associated information, as a process of changing the low-accuracy information.

6. A medical image processing apparatus according to Claim 2,
wherein:
the accepting part accepts a plurality of image data of medical images and a plurality of incidental information thereof;
the associated information is information that associates imaged-site information indicating an imaged site of the medical image as the low-accuracy information, with statistical information indicating a frequency of use of an imaging room for capturing the medical image as the high-accuracy information;
the determining part determines, for each of the plurality of incidental information, whether combination of the imaged-site information and the imaging room conforms to the associated information; and
when two or more of the incidental information are determined not to conform, respectively, the changing part executes a process of interchanging the imaged-site information of the two or more incidental information so as to conform to the associated information, as a process of changing the low-accuracy information.

7. A medical image processing apparatus according to Claim 3, comprising:
an updating part that, when the changing part has executed the process of changing the low-accuracy information, updates the statistical information based on a result of the changing process.

8. A medical image processing apparatus according to Claim 4, comprising:
an updating part that, when the changing part has executed the process of changing the low-accuracy information, updates the statistical information based on a result of the changing process.

9. A medical image processing apparatus according to Claim 5, comprising:
an updating part that, when the changing part has executed the process of changing the low-accuracy information, updates the statistical information based on a result of the changing process.

10. A medical image processing apparatus according to Claim 6, comprising:
an updating part that, when the changing part has executed the process of changing the low-accuracy information, updates the statistical information based on a result of the changing process.

11. A medical image processing apparatus according to Claim 1,
wherein:
the predetermined reference information includes hierarchical information that indicates hierarchies of a plurality of medical information inputted as order information for requesting to capture the medical image; and
when changing the medical information of one hierarchy of the hierarchical information included in the incidental information, the changing part also changes the medical information of lower-level hierarchies than the one hierarchy.

12. A medical image processing apparatus according to Claim 1,
wherein:
the predetermined reference information includes hierarchical information that indicates hierarchies of a plurality of medical information inputted when interpretation of the medical image is performed; and
when changing the medical information of one hierarchy of the hierarchical information included in the incidental information, the changing part also changes the medical information of lower-level hierarchies than the one hierarchy.

13. A medical image processing apparatus according to Claim 1,
wherein:
the accepting part accepts order information for requesting to capture the medical image; and
the changing part includes a comment-information analysis portion that determines whether comment information is included in the order information and, when determining the comment information is included, analyzes the comment information, and the changing part changes the incidental information based on an analysis result of the comment information.

14. A medical image processing apparatus according to Claim 13,
wherein:
the comment information includes string information;
the reference information includes a database storing a meaning of a medical term;
the comment-information analysis portion extracts the medical term included in the database from the string information and obtains the meaning of the extracted medical term from the database; and
the changing part changes the incidental information based on the obtained meaning of the medical term.

15. A medical image processing apparatus according to Claim 13,
wherein:
the comment information includes a schema, which is a schematic view of part of a human body with image information written;
the comment-information analysis portion performs image analysis of the schema; and
the changing part changes the incidental information based on a result of the image analysis.

16. A medical image processing apparatus according to Claim 15,
wherein:
the reference information includes a template of the schematic view of the part of the human body;
the comment-information analysis portion extracts the written image information by calculating a subtraction image between the schema of the accepted order information and the template; and
the changing part changes the incidental information based on the extracted image information.

17. A medical image processing apparatus according to Claim 15,
wherein:
the reference information includes image data of an anatomical drawing of the human body;
the comment-information analysis portion compares the schema of the accepted order information with the anatomical drawing of the human body and determines a region equivalent to the schema in the anatomical drawing of the human body; and
the changing part changes the incidental information based on the determined region.

18. A medical image processing apparatus according to Claim 1,
wherein:
the changing part includes an image analysis portion that analyzes the image data of the medical image accepted by the accepting part and generates the predetermined reference information, and the changing part changes the incidental information based on the generated predetermined reference information.

19. A medical image processing apparatus according to Claim 18,
wherein:
the image analysis portion analyzes the image data of the medical image and extracts a marker image in the medical image as the predetermined reference information; and
the changing part determines an imaged site and/or imaging direction of the medical image based on the extracted marker image, and changes the incidental information based on the determined imaged site and/or imaging direction.

20. A medical image processing apparatus according to Claim 18,
wherein:
the image analysis portion analyzes the image data of the medical image and extracts a background region in the medical image as the predetermined reference information; and
the changing part determines an imaged site and/or imaging direction of the medical image based on the extracted background region, and changes the incidental information based on the determined imaged site and/or imaging direction.

21. A medical image processing apparatus according to Claim 18,
wherein:
the image analysis portion analyzes the image data of the medical image, and generates a pixel-value histogram illustrating a frequency for each pixel value in the medical image, as the predetermined reference information; and
the changing part determines an imaged site and/or imaging direction of the medical image based on the generated pixel-value histogram, and changes the incidental information based on the determined imaged site and/or imaging direction.

22. A medical image processing apparatus according to Claim 18,
wherein:
the image analysis portion analyzes the image data of the medical image, and extracts a region equivalent to part of a human body in the medical image, as the predetermined reference information; and
the changing part determines an imaged site and/or imaging direction of the medical image based on the extracted region, and changes the incidental information based on the determined imaged site and/or imaging direction.

23. A medical image processing apparatus according to Claim 1, comprising:
an outputting part configured to output medical information having been changed by the changing part.

24. A medical image processing apparatus according to Claim 23,
wherein:
the outputting part outputs the medical information having been changed by the changing part and the medical information having not been changed by the changing part, in different display modes.

25. A medical image processing apparatus according to Claim 23,
wherein:
the outputting part outputs the medical information having been changed by the changing part in order of the degree of accuracy, based on the associated information.

26. A medical image processing apparatus according to Claim 23, comprising:
an operating part configured to change the outputted medical information.

27. A medical image processing apparatus according to Claim 26, comprising:
an updating part configured to, when the operating part has changed the medical information has been changed by using the operating part, update the associated information based on the medical information having been changed.

28. A medical image processing apparatus comprising:
an accepting part configured to accept order information for capturing a medical image;
a storing part configured to store the order information accepted by the accepting part; and
a changing part configured to, when the accepting part has accepted new order information, compare the new order information with past order information stored in the storing part, and change the new order information.
